# EUROPEAN PATENT APPLICATION

(11) **EP 4 553 163 A1**
(43) Date of publication of application: **14.05.2025**
(21) Application number: 23208693.4
(22) Date of filing: 09.11.2023
(51) Int. Cl.: C12N 15/82, C12P 7/6409, C12P 7/6463

(54) **FATTY ACID SYNTHASE**

(71) Applicant: Max-Planck-Gesellschaft zur Förderung der Wissenschaften e.V., 80539 München (DE)
(72) Inventor: Chari, Ashwin, 37077 Göttingen (DE); Stark, Holger, 37136 Waake (DE); Singh, Kashish, Cambridge, CB3 1AE (GB)
(74) Representative: Kröncke, Rolf

(57) **Abstract**

The present invention relates to proteins or polypeptides involved in fatty acid synthesis, such as fatty acid synthase (FAS). Said protein comprises one or more polypeptide chains wherein said polypeptide chains comprises one or more subunits having at least one amino acid substitution as defined resulting in altered activity of fatty acid synthesis. The present invention relates further to nucleic acid molecules encoding the proteins or the polypeptide domains as well to host cells containing said nucleic acid molecules, e.g. in form of vectors. The present invention describes further a method for production of fatty acids comprising the cultivation of the host cells according to the present invention as well as the use of the protein according to the present invention or the nucleic acid according to the present invention or the host cell according to the present invention for the production of biofuels, the production of fine chemicals, flavoring compounds, the determination of fungicides and actinobacterial biocides. Moreover, *in silico* methods are described allowing the identification of molecules capable of selectively altering the activity of an enzymatic domain present in the FAS or altering a step of fatty acid synthesis by FAS I comprising determining and modelling molecules interacting under physiological conditions with at least one amino acid in a protein as defined herein. Further, a method of identifying a compound capable of altering at least one of the enzymatic activities of FAS is provided including the step of bringing into contact a candidate compound with the FAS as defined herein and determining whether said candidate demonstrates any interaction with the FAS. Finally, a protein is provided, namely, a mutated ACP which results in alteration of FAS activity.

## Description

The present invention relates to proteins or polypeptides involved in fatty acid synthesis, such as fatty acid synthase (FAS). Said protein comprises one or more polypeptide chains wherein said polypeptide chains comprises one or more subunits having at least one amino acid substitution as defined resulting in altered activity of fatty acid synthesis. The present invention relates further to nucleic acid molecules encoding the proteins or the polypeptide domains as well to host cells containing said nucleic acid molecules, e.g. in form of vectors. The present invention describes further a method for production of fatty acids comprising the cultivation of the host cells according to the present invention as well as the use of the protein according to the present invention or the nucleic acid according to the present invention or the host cell according to the present invention for the production of biofuels, the production of fine chemicals, flavoring compounds, the determination of fungicides and actinobacterial biocides. Moreover, *in silico* methods are described allowing the identification of molecules capable of selectively altering the activity of an enzymatic domain present in the FAS or altering a step of fatty acid synthesis by FAS I comprising determining and modelling molecules interacting under physiological conditions with at least one amino acid in a protein as defined herein. Further, a method of identifying a compound capable of altering at least one of the enzymatic activities of FAS is provided including the step of bringing into contact a candidate compound with the FAS as defined herein and determining whether said candidate demonstrates any interaction with the FAS. Finally, a protein is provided, namely, a mutated or truncated ACP which results in alteration of FAS activity.

### Prior Art

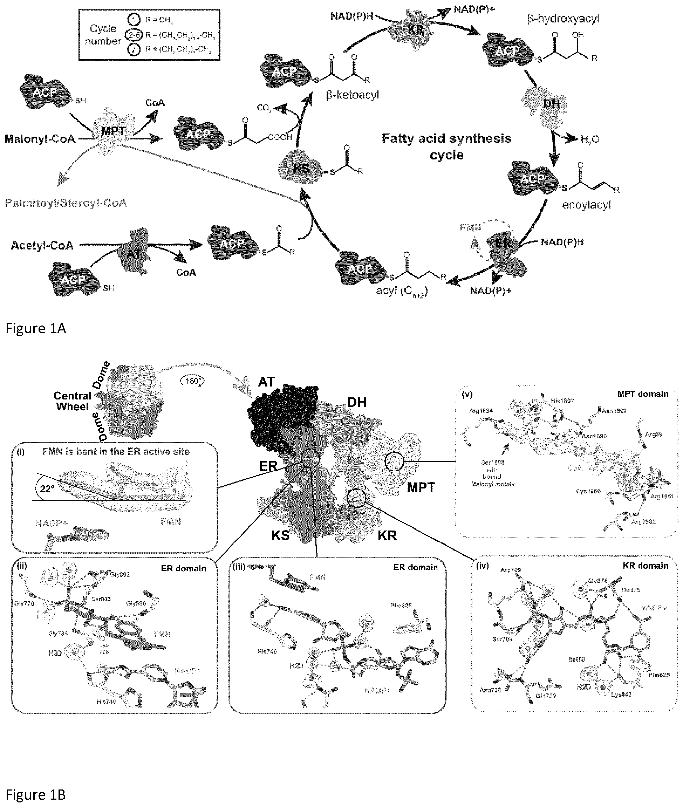

Fatty acids (FAs) biosynthesis follows a sequential and repetitive enzymatic cycle employing acetyl-CoA, malonyl-CoA and NAD(P)H (Figure 1A), Chan, D.I., and Vogel, H.J. (2010), Biochem. J. 430, 1-19. 10.1042/BJ20100462. The acyl carrier protein (ACP) and its post-translational activation on a serine residue by a phosphopantetheine (Ppant) prosthetic group (from CoA) are essential, Chan, D.I., and Vogel, H.J. (2010), see above. FA biosynthesis requires ACP-based active shuttling of biosynthetic acyl intermediates, acetyl-, and malonyl-groups, as covalent adducts to the Ppant-group. In yeast, transacylases transfer the acetyl group of acetyl-CoA (AT domain), or the malonyl group of malonyl-CoA (MPT domain) onto the ACP. The acetyl group is first transferred onto the ketosynthase (KS domain) active site thiol, where it undergoes a condensation reaction with the ACP-bound malonyl group. The resulting Ppant-bound β-ketoacyl-ACP is reduced to β-hydroxyacyl-ACP by the ketoreductase (KR domain), and transferred onto the dehydratase (DH domain). Dehydration of the Ppant-β-hydroxyacyl to the alpha-enoylacyl-ACP, and its reduction by enoyl reductase (ER domain) result in a two-carbon atom elongated alkyl-acyl-ACP. The alkyl-acyl moiety is then transferred to the KS active site thiol, followed by another condensation reaction with MPT-activated malonyl-ACP. The cycle then is repeated until it yields a C16/C18 alkyl-acyl-ACP, which is transacylated to a free CoA molecule by the MPT domain.

Despite this universally conserved biosynthetic cycle, two FA biosynthetic enzyme architectural solutions have evolved. Bacteria, plants, and mitochondria have independent fatty acid synthase (FAS) enzymes, and ACP (type II system), White, S.W., et.al., (2005), Annu. Rev. Biochem. 74, 791-831. 10.1146/annurev.biochem.74.082803.133524. Type II ACP relies on diffusion to interact with different enzymes. Secondly, integrated, large multi-subunit type I FAS systems exist, Schweizer, E., and Hofmann, J. (2004), Microbiol. Mol. Biol. Rev. 68, 501-517. 10.1128/MMBR.68.3.501-517.2004. Type I systems are either 'X'-shaped dimers (α2), typical of metazoan FAS, where each polypeptide contains all catalytic domains connected to the ACP through a flexible linker, or multimeric barrel-like structures where all activities are unified on a single (α₆) in Corynebacteria/Mycobacteria/Nocardia (CMN) bacterial, or two (α₆β₆) polypeptides in fungal FAS. These barrel-like structures are characterized by an equatorial central wheel, with two domes emanating axially from the central wheel. Here, the ACP resides within the FAS barrel, attached by two flexible linkers. The ACP in CMN and fungal FAS is extended by four helices (structural domain), in addition to the canonical four-helix bundle domain of bacterial and metazoan ACP's, Günenc, A.N., et.al., (2022), In Macromolecular Protein Complexes IV: Structure and Function, J. R. Harris and J. Marles-Wright, eds. (Springer International Publishing), pp. 1-33. 10.1007/978-3-031-00793-4_1. Type I FAS systems are thought to be efficient due to compartmentalization of FA biosynthetic reaction chambers, and the spatial proximity of catalytic sites.

Two potential logistical problems arise from the utilization of a common carrier protein to shuttle substrates and intermediates. 1) Hydrophobic ACP cargos need to be shielded from the polar cellular environment when in transit from one enzyme active site to another. In type II ACP, this is achieved by sequestration of bound intermediates into a hydrophobic cleft when in transit. Upon arrival to the next enzyme, the ACP-acyl chain flips out of sequestration and extends into the active site in a "switchblade" mechanism. 2) The common ACP surface and particularly its Ppant-arm are promiscuous, but reduces the number of potential interaction sites to be sampled in specific recognition. In type II FA biosynthetic enzymes, for example, positively charged active site clefts interact with the negatively charged ACP alpha-helices 2 and 3, Chan, D.I., and Vogel, H.J. (2010), see above. However, how these two logistical problems are addressed by type I FAS is unknown, primarily owing to sparse structural insight into ACP shuttling in type I systems.
ACP-compartmentalization, and the rapid transfer between individual enzyme active sites, could make switchblade sequestration unnecessary for fungal FAS. Although, NMR studies on isolated yeast FAS ACP domains indicate the potential for sequestration. Conversely, the larger S. *cerevisiae* ACP engages in more extensive interactions. It binds to the AT and KS enzymatic domains using both canonical and structural ACP domains, Singh, K., et.al., (2020), Cell 180, 1130-1143.e20. 10.1016/j.cell.2020.02.034, while the DH domain only binds the canonical domain. Additionally, it has been reported two conformational states - rotated and non-rotated - adopted by S. *cerevisiae* β-subunits that are associated with distinct spatial positions of ACP domains, Singh, K., et.al., (2020), see above. The non-rotated conformation correlated with ACP-KS domain binding, while the rotated was consistent with ACP-AT domain binding, additionally stabilized by a newly discovered gamma-subunit. FA biosynthesis requires a cyclic, sequential, and ordered interaction of the ACP with six catalytic domains in total (Figure 1A). Two large-scale conformational rearrangements alone are probably insufficient for all these ACP interactions in the course of FA biosynthesis.

In view of the above, it is desired and addressed to identify relevant domains and amino acids involved in the enzymatic activity of FAS including the catalytic domain but also identifying interacting domains. In particular, interaction of the ACP with the active sites present in the FAS allows to identify suitable molecules including mutated ACP molecules resulting in a manipulation of the FAS activity. This allows to provide improved means and methods for producing fatty acids in microorganisms as on the one hand, in particular, fatty acids that are suitable for biofuel production, fine chemicals, and other compounds. In a second aspect, this allows to identify compounds suitable as fungicides or actinobacterial biocides.

### Summary of the invention

According to the present invention the above objects have been solved by a polypeptide or protein involved in fatty acid synthesis in accordance with the claims. Namely, in a first aspect, a polypeptide or protein is provided, said protein comprises one or more polypeptide chains, wherein said polypeptide chain(s) comprise
i) one or more subunits comprising the amino acid sequence of
   SEQ ID No. 1 (ER domain);
   SEQ ID No. 2 (AT domain);
   SEQ ID No. 3 (KS domain);
   SEQ ID No.4 (DH domain);
   SEQ ID No.5 (KR domain);
   SEQ ID No.6 (MPT domain); and/or
   SEQ ID No. 7 (ACP domain);
ii) having at least one amino acid substitution in the ER domain at a position corresponding to 32-140, 159-190, 211-220, 239-250, 288-329, 381-390, 450-480, 500-505 of the amino acid sequence of SEQ ID No. 1; and/or at least one amino acid substitution in the AT domain at a position corresponding 5-141, 162-166, 273-277, 362-368, 291-430, 542-554 of the amino acid sequence of SEQ ID No. 2; and/or at least one amino acid substitution in the KS domain at a position corresponding to amino acids 68-131of the amino acid sequence of SEQ ID No. 3; and/or an amino acid substitution in the DH domain at a position 189-310 corresponding to of the amino acid sequence of SEQ ID No. 4; and/or an amino acid substitution in the KR domain at a position corresponding to 36-66, 176-187, 353-417, 442-460, 498-520, 543-560, 602-643 of the amino acid sequence of SEQ ID No. 5; and/or at least one amino acid substitution in the MPT domain at a position corresponding to 1-30, 48-170, 242-276, 293-306, 325-335, 353-358, 396-415 of SEQ ID No. 6; and/or at least one amino acid substitution in the ACP domain at a position corresponding to 5-11, 20-25, 34-105, 135-147 of SEQ ID No. 7;
wherein the amino acid sequence comprising at least one amino acid substitution has at least 70 %, or preferably at least 80 % or 90 % or 95 % sequence identity to the respective amino acid sequence of SEQ ID No. 1 and/or of SEQ ID. No. 2 and/or SEQ ID No. 3 and/or SEQ ID No. 4 and/or SEQ ID No. 5 and/or SEQ ID No. 6 and/or SEQ ID No. 7; resulting in altered activity of fatty acid synthesis.

In a further aspect, the present invention is directed to nucleic acid molecules encoding for a protein or for a polypeptide according to the present invention, preferably further comprising vector nucleic acid sequences, more preferably expression vector sequences and/or comprising promotor nucleic acid sequences and terminator nucleic acid sequences, and/or comprising other regulatory nucleic acid sequences and/or in the nucleic acid molecule preferably comprises dsDNA, ssDNA, cDNA, LNA, PNA, CNA, RNA or mRNA or combinations thereof.

Moreover, a host cell containing a nucleic acid molecule according to the present invention and, preferably expressing said nucleic acid molecule, wherein said host cell is preferably selected from a bacterial cell, a fungal cell and an algal cell is provided.

Further, a method for production of fatty acid cells is disclosed comprising a cultivation step of host cells according to the present invention. In particular, the method is a method for the production of fatty acids having a defined size, e.g. short fatty acids (C6 to C12) as well as long fatty acids (C16 to C18).

Moreover, the use of the protein according to the present invention, the nucleic acid according to the present invention or a host cell according to the present invention is provided, the use includes the production of biofuel, the production of fine chemicals, the production of flavoring substances. Further, the use refers to the determination of fungicides and actinobacterial biocides.

Another aspect of the present invention relates *in silico* methods for identifying molecules. Hence, the *in silico* method allows identifying molecules capable of selectively altering a step of fatty acid synthesis by FAS I comprising determining and modelling molecules interacting under physiological conditions with at least one amino acid as defined herein, namely, at least one amino acid in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7.

In addition, an *in silico* method of identifying molecules capable of selectively altering the activity of an enzymatic domain present in the FAS I whereby the molecules determined and modelled showed interaction under physiological conditions with at least one amino acid in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7 resulting in altering at least enzymatic activity in the FAS.

In addition, a method of identifying a compound capable of altering at least one of the enzymatic activities of FAS I is provided, said method comprising bringing into contact a candidate compound with the FAS I and determining whether said candidate compound (i) interacting with at least one amino acid in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7 and/or (ii) is a competitor of ACP in interacting with at least one amino acid in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7.

In a further aspect, a protein is provided having mutations in SEQ ID No. 7, said mutations are at least one amino acid at a position corresponding to 5-11, 20-25, 34-105, 135-147 of SEQ ID No. 7.

In an aspect, fungicides or actinobacterial biocides are provided wherein its effective mechanism is with FAS I of fungi and of actinobacteria.

### Detailed description of the present invention

In a first aspect, the present invention relates to a protein (polypeptide) involved in fatty acid synthesis, said protein comprises one or more polypeptide chains, wherein said polypeptide chain(s) comprise
i) one or more subunits comprising the amino acid sequence of
   SEQ ID No. 1 (ER domain);
   SEQ ID No. 2 (AT domain);
   SEQ ID No. 3 (KS domain);
   SEQ ID No.4 (DH domain);
   SEQ ID No.5 (KR domain);
   SEQ ID No.6 (MPT domain); and/or
   SEQ ID No. 7 (ACP domain);
ii) having at least one amino acid substitution in the ER domain at a position corresponding to 32-140, 159-190, 211-220, 239-250, 288-329, 381-390, 450-480, 500-505 of the amino acid sequence of SEQ ID No. 1; and/or at least one amino acid substitution in the AT domain at a position corresponding 5-141, 162-166, 273-277, 362-368, 291-430, 542-554 of the amino acid sequence of SEQ ID No. 2; and/or at least one amino acid substitution in the KS domain at a position corresponding to amino acids 68-131of the amino acid sequence of SEQ ID No. 3; and/or an amino acid substitution in the DH domain at a position 189-310 corresponding to of the amino acid sequence of SEQ ID No. 4; and/or an amino acid substitution in the KR domain at a position corresponding to 36-66, 176-187, 353-417, 442-460, 498-520, 543-560, 602-643 of the amino acid sequence of SEQ ID No. 5; and/or at least one amino acid substitution in the MPT domain at a position corresponding to 1-30, 48-170, 242-276, 293-306, 325-335, 353-358, 396-415 of SEQ ID No. 6; and/or at least one amino acid substitution in the ACP domain at a position corresponding to 5-11, 20-25, 34-105, 135-147 of SEQ ID No. 7;
wherein the amino acid sequence comprising the at least one amino acid substitution has at least 70 %, or preferably at least 80 % or 90 % or 95 % sequence identity to the respective amino acid sequence of SEQ ID No. 1 and/or of SEQ ID. No. 2 and/or SEQ ID No. 3 and/or SEQ ID No. 4 and/or SEQ ID No. 5 and/or SEQ ID No. 6 and/or SEQ ID No. 7; resulting in altered activity of fatty acid synthesis.
Unless otherwise indicated herein, the term "FAS" refers to the FAS I complex of yeast or actinobacteria.

The present inventors identified by structural snapshots of the FAS I with ACP the relevant intermediate stages in fatty acid (FA) biosynthesis revealing relevant amino acid residues present in the enzymes building the FAS I complex as well as in the ACP molecule allowing to interrogate fungal FAS FA biosynthesis mechanisms at atomic detail for biotechnological expiration.

The amino acid regions identified above have been recognized as relevant region/positions in ACP FAS I interaction. Changing at least one amino acid at the relevant positions would result in change of interaction between ACP and the respective enzyme. In this connection it is noted that the present inventors recognized also regions of adjacent enzymes fostering the ACP binding and interaction in the enzymatic site of a different enzyme. Changing at least one of the amino acids either present in the enzyme or the ACP results in immediate or indirect influence on enzymatic activity eventually resulting in a change of productivity of the FAS I complex.

Depending on the affected enzyme, the outcome will be a change of the fatty acid produced, e. g. on the length of the fatty acid molecule. In some cases, the amino acid and regions identified allow to determine compounds and molecules inhibiting the activity of at least one of the enzymes thus being toxic to a fungi or actinobacteria.

Namely, identifying the relevant positions of the FAS I and the ACP allows to develop appropriate molecules representing suitable inhibitors of the same.

As used therein, the term "involved in fatty acid synthesis" refers to a protein or polypeptide relevant for the synthesis of fatty acid molecules in a cell. In particular, the protein involved in fatty acid synthesis is a member of the FAS I complex of yeast or actinobacteria or the ACP protein.

That is, in an aspect, the protein involved in fatty acid synthesis can be the protein or polypeptide of ACP.

The term "altered activity of fatty acid synthesis" refers to a change of the synthesised products, e. g. length of the fatty acid molecules, or a change in the enzymatic reaction rate of at least one of the enzymes present in the FAS I complex. Further, the alteration may be due to changes in the assembly and/or architecture of the FAS I complex. Alterations may be an inhibition or an increase or decrease of the enzymatic activity or the enzymatic reaction. The alteration may be due to change of the activity of one or more of the enzymes present in FAS I. Eventually, the alteration may result in an increase or decrease of selective production of specific fatty acids and fatty acid derivatives.

The activity can be influenced for example by increasing or decreasing the affinity and avidity between the ACP and the interacting enzymatic structural regions of the FAS I complex.

In addition, as used herein, the term "interacting" refers to a change or affinity and avidity between different regions of the FAS I or between at least one region of FAS I and ACP. The change in to at least one amino acid residue of the FAS I complex or ACP of amino acid residues or regions of amino acid residues as defined herein. In particular, the interaction refers to an increase in affinity and avidity between binding partners involved. For example, the increase in affinity and avidity results in reduction of the enzymatic reaction rate of said enzyme being a member of the FAS I complex. The mode of interaction can involve at least one of attractive forces: covalent (i.e. forming a chemical bond), forming a salt bridge by electrostatic interactions, a hydrogen bond- involving a hydrogen bond donor and acceptor, or hydrophobic association (mediated through van der Waals forces).

As referred herein, the term "physiological conditions" identifies the conditions present in a cell, e.g. a host cell or of fungal cells or actinobacterial cells resulting in an interaction between the molecules or compounds and the amino acid residues as defined herein.

As used herein, the term, "percent (%) identical" refers to sequence identity between two amino acid sequences. Identity can be determined by comparing a position or sequences, which may be aligned for the purpose of comparison. When an equivalent position in the compared sequence is occupied by the same amino acid, the molecules are considered to be identical at that position.

As used herein, the term "functional equivalent" refers to amino acid sequences that are not 100 % identical to the amino acid sequence mentioned herein comprising amino acid additions and/or insertions and/or deletions and/or substitutions and/or exchanges which do not alter or changes the activity or function of the protein as compared to the protein or polypeptide as defined herein. For example, a functional equivalent encompasses an amino acid sequence with conservative amino acid substitutions or smaller deletions and/or insertions as long as these modifications do not substantially affect the *in vitro* and/or *in vivo* fatty acid synthase enzymatic activity.

Generally, a person skilled in the art is aware of the fact that some amino acids exchanges in the amino acid sequence of a protein or a polypeptide do not influence on the (secondary or tertiary) structure, function and/or activity of that protein. Amino acid sequences with such "natural" amino acid exchanges as compared to the amino acid sequences disclosed herein form within the scope of the present invention.

Further, the sequences may be optimized by protein design software including software like Rosetta, RosettaFold, AlphaFold2, Gromacs, Swiss-Modeller, Modeller, Quark, Prime, Phyre, Homelette, AMBER, Abalone, ADF, Avogadro, CHARMM, BOSS, CP2k, Desmond, Discovery Studio, GROMOS, LAMMPS, MacroModel, MAPS, Materials Studio, MBN Explorer, MDynaMix, MOE, OpenMM, Orac, NAMD + VMD, NWChem, Protein Local Optimization Program, Q, Quantum ATK, SAMSON, Scigress, TINKER, TeraChem, Yasara for adapting the specific proteins or polypeptides to host cells present in the same. In addition, suitable substitutions or the at least one amino acid as defined herein can be determined with appropriate protein design software accordingly.

The term "competitor" as used herein refers to molecules which prevent or interact with substrate molecules to bind to active sites of enzymes, namely, compete for binding to the same binding site. The competitor, e. g. an altered ACP molecule having a modified or mutated amino acid sequence compete with natural ACP for the same binding site, thus, interacting with and/or interrupting the enzymatic reaction. The protein or polypeptide types of the present invention, in particular, the type I FAS variants, have an *in vitro* and/or *in vivo* fatty acid synthase I enzymatic activity.

In an embodiment, the at least one amino acid substitution is present in the amino acid sequence of SEQ ID No. 7 representing the amino acid sequence of ACP.

The present inventors recognized that at least one amino acid in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7 are involved in enzymatic reaction and/or binding of ACP in its different forms with the active site of an enzyme. That is the relevance is not only related to be part of the catalytic site of the relevant enzyme but the adjacent region of amino acids may be relevant for interaction and binding of ACP enabling the enzymatic reaction accordingly. For example, as shown herein and discussed below, structure elements in distance from the active sites are generally involved in ACP binding. Further, the yeast specific ACP structural domain mediates interactions also with the adjacent regions of other enzymes, e.g. as discussed below. The canonical ACP domain is universally required for interaction with all enzymatic domains of FAS. A particular relevance is with the Ser180 corresponding to Ser44 of SEQ ID No 7.

In an aspect, affinity and avidity based on various types of interaction between ACP and structure elements of the FAS I complex are relevant for binding of ACP and binding force between ACP and the structural element. The binding force is of course of particular relevance for the enzymatic reaction rate and the switching of ACP from one active site to another. Hence, motion of ACP in the FAS I complex can be changed and, in some cases, disrupted. When the enzymatic cascade is essentially interrupted, an inhibition of the FAS I complex is present accordingly.

In an embodiment of the present invention, the relevant amino acids are at least one amino acid as follows: at least one amino acid in the ER domain at a position corresponding to 40-45, 65-70, 92-109, 126-140, 160-170, 175-188, 314-324, 290-301, 467-477 of the amino acid sequence of SEQ ID No. 1; and/or at least one amino acid in the AT domain at a position corresponding 163, 274, 364, 401, 403, 405, 406, 428 of the amino acid sequence of SEQ ID No. 2; and/or at least one amino acid in the KS domain at a position corresponding to amino acids 95-102, 109-130 of the amino acid sequence of SEQ ID No. 3; and/or an amino acid in the DH domain at a position 198 corresponding to of the amino acid sequence of SEQ ID No. 4; and/or an amino acid in the KR domain at a position corresponding to 39-62, 178-184, 354-364, 372-392, 409-413, 545-557, 603-642 of the amino acid sequence of SEQ ID No. 5; and/or at least one amino acid in the MPT domain at a position corresponding to 20-28, 48-64, 81-96, 102-110 of SEQ ID No. 6; and/or at least one amino acid in the ACP domain at a position corresponding to 40-49, 51-70, 72-105of SEQ ID No. 7.

That is, with protein or peptide according to the present invention comprises at least one amino acid substitution in the ER domain at a position corresponding to 40-45, 65-70, 92-109, 126-140, 160-170, 175-188, 314-324, 290-301, 467-477 of the amino acid sequence of SEQ ID No. 1; and/or at least one amino acid substitution in the AT domain at a position corresponding 163, 274, 364, 401, 403, 405, 406, 428 of the amino acid sequence of SEQ ID No. 2; and/or at least one amino acid substitution in the KS domain at a position corresponding to amino acids 95-102, 109-130 of the amino acid sequence of SEQ ID No. 3; and/or an amino acid substitution in the DH domain at a position 198 corresponding to of the amino acid sequence of SEQ ID No. 4; and/or an amino acid substitution in the KR domain at a position corresponding to 39-62, 178-184, 354-364, 372-392, 409-413, 545-557, 603-642 of the amino acid sequence of SEQ ID No. 5; and/or at least one amino acid substitution in the MPT domain at a position corresponding to 20-28, 48-64, 81-96, 102-110 of SEQ ID No. 6; and/or at least one amino acid substitution in the ACP domain at a position corresponding to 40-49, 51-70, 72-105of SEQ ID No. 7.

Of note, the terms "protein" and "polypeptide" are used interchangeably herein unless otherwise indicated.

In an embodiment, the amino acid relevant and recognized according to the present invention are shown in table I:

**Table I**

| Polypetide | SEQ ID No. | Position SEQ ID | Position alpha or beta unit | |
|---|---|---|---|---|
| ER | 1 | 40 | 596 | Beta subunit |
| | | 42 | 598 | |
| | | 66 | 622 | |
| | | 68 | 624 | |
| | | 94 | 650 | |
| | | 97 | 653 | |
| | | 98 | 654 | |
| | | 99 | 655 | |
| | | 100 | 656 | |
| | | 101 | 657 | |
| | | 103 | 659 | |
| | | 104 | 660 | |
| | | 108 | 664 | |
| | | 127 | 683 | |
| | | 128 | 684 | |
| | | 129 | 685 | |
| | | 130 | 686 | |
| | | 131 | 687 | |
| | | 133 | 689 | |
| | | 134 | 690 | |
| | | 137 | 693 | |
| | | 140 | 696 | |
| | | 150 | 706 | |
| | | 152 | 708 | |
| | | 153 | 709 | |
| | | 155 | 711 | |
| | | 156 | 712 | |
| | | 159 | 715 | |
| | | 177 | 733 | |
| | | 180 | 736 | |
| | | 182 | 738 | |
| | | 184 | 740 | |
| | | 186 | 742 | |
| | | 214 | 770 | |
| | | 244 | 800 | |
| | | 246 | 802 | |
| | | 247 | 803 | |
| | | 248 | 804 | |
| | | 291 | 847 | |
| | | 293 | 849 | |
| | | 294 | 850 | |
| | | 295 | 851 | |
| | | 296 | 852 | |
| | | 300 | 856 | |
| | | 317 | 873 | |
| | | 318 | 874 | |
| | | 319 | 875 | |
| | | 320 | 876 | |
| | | 321 | 877 | |
| | | 384 | 940 | |
| | | 452 | 1008 | |
| | | 454 | 1010 | |
| | | 455 | 1011 | |
| | | 467 | 1023 | |
| | | 468 | 1024 | |
| | | 470 | 1026 | |
| | | 471 | 1027 | |
| | | 474 | 1030 | |
| | | 475 | 1031 | |
| | | 477 | 1033 | |
| | | 503 | 1059 | |
| AT | 2 | 163 | 163 | Beta subunit |
| | | 274 | 274 | |
| | | 364 | 364 | |
| | | 401 | 401 | |
| | | 403 | 403 | |
| | | 405 | 405 | |
| | | 406 | 406 | |
| | | 428 | 428 | |
| KS | 3 | 96 | 1066 | Alpha subunit |
| | | 98 | 1068 | |
| | | 99 | 1069 | |
| | | 100 | 1070 | |
| | | 101 | 1071 | |
| | | 109 | 1079 | |
| | | 110 | 1080 | |
| | | 112 | 1082 | |
| | | 113 | 1083 | |
| | | 117 | 1087 | |
| | | 118 | 1088 | |
| | | 120 | 1090 | |
| | | 121 | 1091 | |
| | | 122 | 1092 | |
| | | 125 | 1095 | |
| | | 126 | 1096 | |
| | | 129 | 1099 | |
| | | 130 | 1100 | |
| DH | 4 | 198 | 1439 | Beta subunit |
| KR | 5 | 42 | 369 | Alpha subunit |
| | | 45 | 372 | |
| | | 48 | 375 | |
| | | 49 | 376 | |
| | | 52 | 379 | |
| | | 53 | 380 | |
| | | 57 | 384 | |
| | | 58 | 385 | |
| | | 61 | 388 | |
| | | 179 | 506 | |
| | | 181 | 508 | |
| | | 182 | 509 | |
| | | 183 | 510 | |
| | | 355 | 682 | |
| | | 358 | 685 | |
| | | 360 | 687 | |
| | | 361 | 688 | |
| | | 362 | 689 | |
| | | 372 | 699 | |
| | | 379 | 706 | |
| | | 380 | 707 | |
| | | 381 | 708 | |
| | | 382 | 709 | |
| | | 384 | 711 | |
| | | 386 | 713 | |
| | | 391 | 718 | |
| | | 410 | 737 | |
| | | 411 | 738 | |
| | | 412 | 739 | |
| | | 444 | 771 | |
| | | 448 | 775 | |
| | | 450 | 777 | |
| | | 451 | 778 | |
| | | 452 | 779 | |
| | | 453 | 780 | |
| | | 456 | 783 | |
| | | 458 | 785 | |
| | | 460 | 787 | |
| | | 500 | 827 | |
| | | 502 | 829 | |
| | | 512 | 839 | |
| | | 516 | 843 | |
| | | 545 | 872 | |
| | | 548 | 875 | |
| | | 549 | 876 | |
| | | 552 | 879 | |
| | | 553 | 880 | |
| | | 554 | 881 | |
| | | 556 | 883 | |
| | | 603 | 930 | |
| | | 609 | 936 | |
| | | 610 | 937 | |
| | | 617 | 944 | |
| | | 621 | 948 | |
| | | 625 | 952 | |
| | | 628 | 955 | |
| | | 632 | 959 | |
| | | 636 | 963 | |
| | | 642 | 969 | |
| MPT | 6 | 2 | 2 | Alpha subunit |
| | | 21 | 21 | |
| | | 22 | 22 | |
| | | 25 | 25 | |
| | | 26 | 26 | |
| | | 49 | 49 | |
| | | 50 | 50 | |
| | | 51 | 51 | |
| | | 52 | 52 | |
| | | 53 | 53 | |
| | | 55 | 55 | |
| | | 56 | 56 | |
| | | 58 | 58 | |
| | | 59 | 59 | |
| | | 62 | 62 | |
| | | 83 | 83 | |
| | | 87 | 87 | |
| | | 93 | 93 | |
| | | 94 | 94 | |
| | | 103 | 1667 | Beta subunit |
| | | 106 | 1670 | |
| | | 107 | 1671 | |
| | | 109 | 1673 | |
| | | 243 | 1807 | |
| | | 244 | 1808 | |
| | | 248 | 1812 | |
| | | 267 | 1831 | |
| | | 270 | 1834 | |
| | | 274 | 1838 | |
| | | 294 | 1858 | |
| | | 297 | 1861 | |
| | | 305 | 1869 | |
| | | 326 | 1890 | |
| | | 328 | 1892 | |
| | | 329 | 1893 | |
| | | 331 | 1895 | |
| | | 332 | 1896 | |
| | | 354 | 1918 | |
| | | 398 | 1962 | |
| | | 400 | 1964 | |
| | | 403 | 1967 | |
| | | 405 | 1969 | |
| | | 406 | 1970 | |
| | | 412 | 1976 | |
| | | 413 | 1977 | |
| | | 424 | 1988 | |
| ACP | 7 | 6 | 142 | Alpha subunit |
| | | 8 | 144 | |
| | | 10 | 146 | |
| | | 21 | 157 | |
| | | 22 | 158 | |
| | | 24 | 160 | |
| | | 35 | 171 | |
| | | 37 | 173 | |
| | | 41 | 177 | |
| | | 42 | 178 | |
| | | 43 | 179 | |
| | | 44 | 180 | |
| | | 45 | 181 | |
| | | 46 | 182 | |
| | | 47 | 183 | |
| | | 48 | 184 | |
| | | 49 | 185 | |
| | | 51 | 187 | |
| | | 52 | 188 | |
| | | 53 | 189 | |
| | | 55 | 191 | |
| | | 56 | 192 | |
| | | 57 | 193 | |
| | | 58 | 194 | |
| | | 59 | 195 | |
| | | 60 | 196 | |
| | | 61 | 197 | |
| | | 62 | 198 | |
| | | 63 | 199 | |
| | | 64 | 200 | |
| | | 66 | 202 | |
| | | 67 | 203 | |
| | | 68 | 204 | |
| | | 69 | 205 | |
| | | 72 | 208 | |
| | | 76 | 212 | |
| | | 78 | 214 | |
| | | 79 | 215 | |
| | | 80 | 216 | |
| | | 81 | 217 | |
| | | 82 | 218 | |
| | | 84 | 220 | |
| | | 86 | 222 | |
| | | 87 | 223 | |
| | | 88 | 224 | |
| | | 91 | 227 | |
| | | 95 | 231 | |
| | | 98 | 234 | |
| | | 99 | 235 | |
| | | 100 | 236 | |
| | | 101 | 237 | |
| | | 103 | 239 | |
| | | 104 | 240 | |
| | | 136 | 272 | |
| | | 138 | 274 | |
| | | 139 | 275 | |
| | | 140 | 276 | |
| | | 142 | 278 | |
| | | 143 | 279 | |
| | | 144 | 280 | |
| | | 145 | 281 | |
| | | 146 | 282 | |

As discussed above, the present invention provides a nucleic acid molecule, encoding for a protein or polypeptide according to the present invention. The present invention provides nucleic acid molecules which comprises the respective nucleotide exchange which lead to the amino acid substitution(s) of the present invention.

In one embodiment, the nucleic acid molecules of the present invention further comprises:
i) vector nucleic acid sequences, preferably expression vector sequences; and/or
ii) promoter nucleic acid sequences and terminator nucleic acid sequences; and/or
iii) other regulatory nucleic acid sequences.

In a further embodiment, the nucleic acid molecule of the present invention comprises dsDNA, ssDNA, cDNA, LNA, PNA, CNA, RNA or mRNA or combinations thereof.

In an embodiment, the nucleic acid molecules according to the present invention preferably comprises nucleic acid sequences which are identical with the natural occurring nucleic acid sequences or are non-current optimized for the use in a host cell accept for the addition of the amino acid substitution(s) according to the invention. In the nucleic acid molecule use according to the present invention is preferably a nucleic acid expression construct. Nucleic acid expression constructs according to the present invention are expression cassettes comprising a nucleic acid molecule according to the invention, or expression vectors comprising a nucleic acid molecule according to the invention or an expression corset. Typically, the nucleic acid expression constructs comprise regulatory sequences, such as promoter and terminator sequences, which are operatively linked with a nucleic sequence encoding for the polypeptide(s) of the invention.

In a further aspect, a host cell containing a nucleic acid molecule according to the present invention and preferably expression said nucleic acid molecule are provided.

The host cell may be a host cell selected from a bacterial cell, a fungal cell, an actinobacterial cell and an algal cell, or eubacteria.
For example, in case of eubacteria E.coli or B. subtillis may be used.

In an embodiment, the host cell is a non-oleaginous yeast. For example, suitable non-oleaginous yeasts include a member of a genus selected from the group of saccharomyces, Schizosaccharomyces, like saccharomyces cerevisiae, but also Pichia pastoris or Kluyveromyces lactis, and Ustilago maydis. In case of oleaginous yeast which represents suitable host cells, the oleaginous genus may be selected from candida, Rhodosporidium, Yarrowia, Cryptococcus, Rhodotorula, Lipomyces, and Trichosporon. A particularly suitable example of a host cell is Yarrowia lipolytica (Y. lipolytica). Further, the host cell may be an actinobacteria, like mycobacteria.

Suitable algae cells may be a member of a genius selected from Chlamydomonas, Chlorella, Haematococcus, Dunaliella, Nanochloroypsis, thalassiorao, phaerodactylum, Porphyridium or scenedesmus.

For example, in case of the use of a host cell according to the present invention for the production of biofuels, the genus of Yarrowia, like Yarrowia lipolytica is used.

In a further aspect, the present invention relates to a method for the production of fatty acids. The method comprises the step of cultivation of host cells according to the present invention and/or the expression of a protein involved in fatty acid synthesis according to the present invention, thus, allowing fatty acid synthesis accordingly. For example, it is possible to increase the production of short fatty acid derivatives of C6 to C12 fatty acids, like C6, C8, C10, C12 fatty acids. Said fatty acids may be present as fatty acids or as ester or thioester, containing said fatty acids. Further, it is possible to obtain an elevated overall production of long fatty acids, namely fatty acids of C16 and/or C18 in length. Depending on the alteration on the enzymatic activity of the subunits present in the FAS I complex, the product distribution of the fatty acids produced by the FAS I complex can be influenced accordingly.

The method includes a method for the production of fatty acids, comprising the step of expressing a nucleic acid molecule according to the present invention, preferably in a host cell according to the present invention, or of the protein or polypeptide according to the present invention.

In an embodiment of the method according to the present invention, the method is for the production of short chain (C6 to C12) fatty acids wherein the protein or polypeptide contains preferably the following substitutions: Met56Val, Met56lle, Val26Leu, Val26Ile, Lys62Arg, Gln58Glu, Lys64Arg, Lys83Arg, Asn326Asp, Asn326Gln, Asn326Glu, Asn328Asp, Asn328Gln, Asn328Glu, Gln333His, Gln333Glu, Arg398Glu, Arg398His, His413Asp, His413Asn, His413Glu, His413Gln, His413Tyr, His413Arg in SEQ ID No.6; or Lys37Arg, Lys37Glu, Lys37Gln, Gly42his, Gly42Tyr, Gly42Phe, Asn48Asp, Asn48Gln, Asn48Glu, Asn48His, Asn48Arg, Asn48Tyr, Thr45Met, Thr45His, Thr45Asn, Thr45Asp, Thr45Gln, Thr45Glu, Leu51Arg, Leu51Tyr, Lys64Glu, Lys64Arg, Glu67Asn, Glu67Asp, Glu67Gln, Glu67His, Glu67Tyr, Ser98Tyr, Ser98His, Ser98Glu, Ser98Gln, Ser98Arg, Ser98Lys, Ser99Tyr, Ser99His, Ser99Glu, Ser99Gln, Ser99Arg, Ser99Lys in SEQ ID No.7, or expressing a nucleic acid molecule coding for said protein or polypeptide, preferably in a host cell according to the present invention. The specific mutations mentioned have influence on the MPT/ACP interaction, eventually resulting in the increased production of short fatty acid derivatives of C6 to C12 fatty acids.

In a further aspect, the present invention relates to a use of the protein or polypeptide according to the present invention, the nucleic acid according to the present invention and/or the host cell according to the present invention for various purposes. For example, the use includes a production of biofuels. As mentioned above, biofuels with short fatty acids are desired. Further, the production of fine chemicals using the protein or polypeptides according to the present invention, the nucleic acid molecules according to the present invention and/or the host cells according to the present invention is envisaged.

Further, the use includes the determination of fungicides and/or biocides. Namely, interfering with the activity of the FAS I complex, in particular, inhibiting the activity of the FAS I complex allows to provide respective biocides, namely, bacterial biocides, as well as fungicides. That is, beside suitable inhibitors of fungi, it is also envisaged that biocides including mycobacterial biocides can be provided. In this connection, the use also includes the determination of agents that reduce actinobacterial mycolic acid production. Namely, as said with respect to the mycobacterial biocides, also reduction of actinobacterial mycolic acid production, eventually, allowing to decrease mycobacterial or actinobacterial action. Not to be bound by theory it is submitted that reducing the actinobacterial mycolic acid production which is affected by the FAS I complex, the cell wall components are weakened or depleted, thus, destruction of the actinobacteria, e. g. mycobacteria is enhanced.

In a further aspect, *in silico* methods are provided. Namely, in a further aspect an in silico method of identifying molecules capable of selectively altering a step of fatty acid synthesis by FAS I comprising determining and modelling molecules interacting under physiological conditions with at least one amino acid in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7 is provided.

In a further aspect, an *in silico* method of identifying molecules capable of selectively altering the activity of an enzymatic domain present in the FAS whereby the molecules determined and modelled show interaction under physical conditions with at least one amino acid as in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7; resulting in altering at least enzymatic activity in the FAS is disclosed herein.

In an embodiment, the method refers to at least one amino acid in the ER domain at a position corresponding to 40-45, 65-70, 92-109, 126-140, 160-170, 175-188, 314-324, 290-301, 467-477 of the amino acid sequence of SEQ ID No. 1; and/or at least one amino acid substitution in the AT domain at a position corresponding 163, 274, 364, 401, 403, 405, 406, 428 of the amino acid sequence of SEQ ID No. 2; and/or at least one amino acid substitution in the KS domain at a position corresponding to amino acids 95-102, 109-130 of the amino acid sequence of SEQ ID No. 3; and/or an amino acid substitution in the DH domain at a position 198 corresponding to of the amino acid sequence of SEQ ID No. 4; and/or an amino acid substitution in the KR domain at a position corresponding to 39-62, 178-184, 354-364, 372-392, 409-413, 545-557, 603-642 of the amino acid sequence of SEQ ID No. 5; and/or at least one amino acid substitution in the MPT domain at a position corresponding to 20-28, 48-64, 81-96, 102-110 of SEQ ID No. 6; and/or at least one amino acid substitution in the ACP domain at a position corresponding to 40-49, 51-70, 72-105of SEQ ID No. 7.

Preferably, the amino acid are amino acids as shown in table 1 above.

That is, the *in silico* method according to a first embodiment and to further embodiments, refers to identifying molecules capable of selectively altering a synthesis by FAS I complex. The method includes the application of a suitable software based on the data of the FAS I complex identified. The respective data set are available under the respective EMDB ID or under PDB ID as shown in table 2.

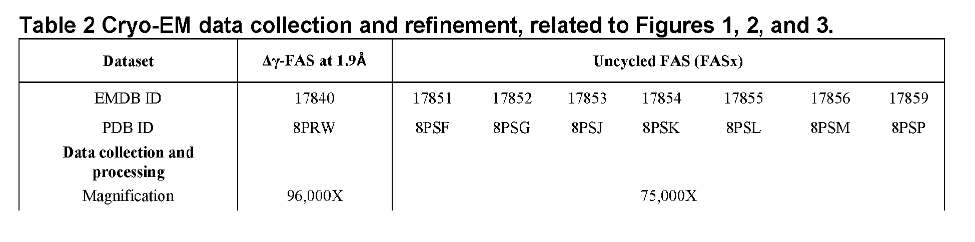

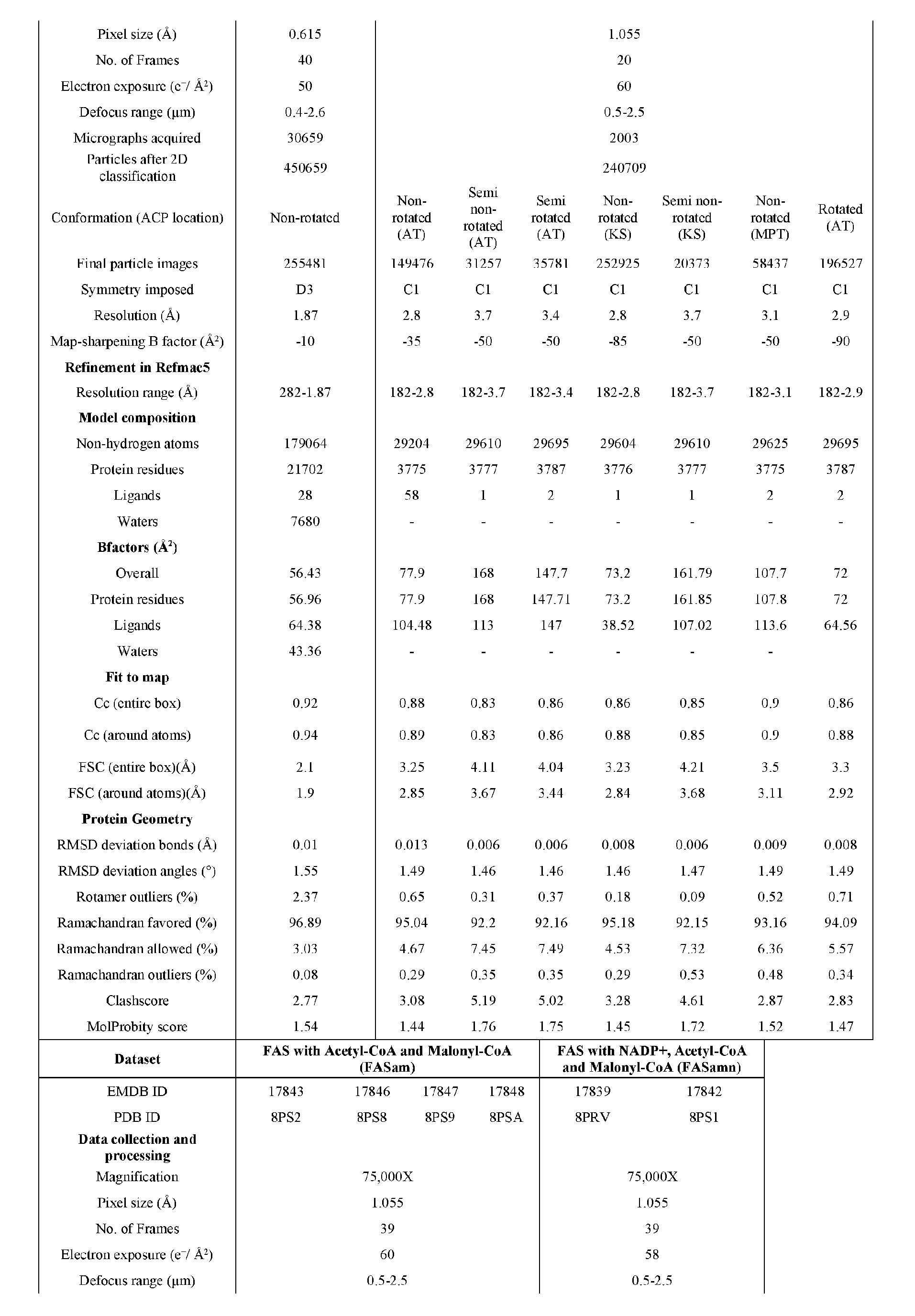

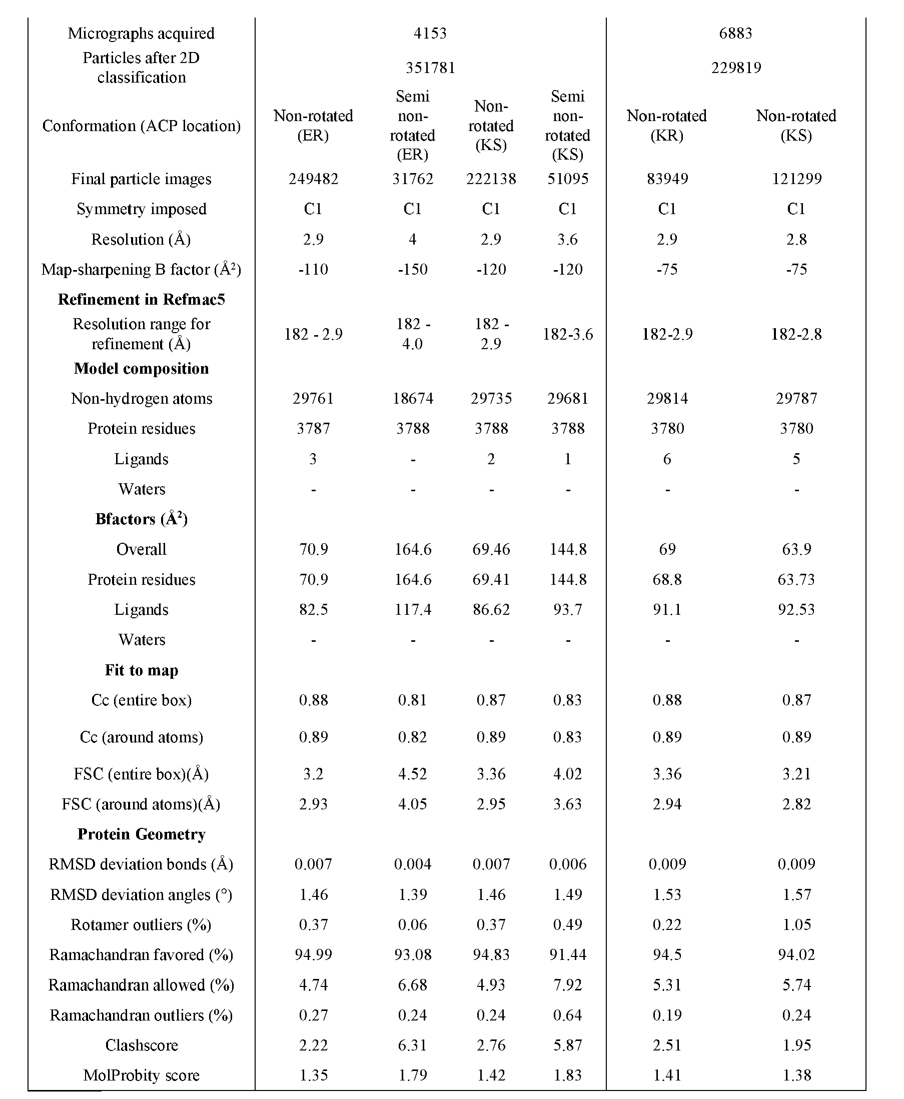

Respective molecules interacting under physiological conditions are determined and modelled by suitable software tools for design accordingly. As noted above, interacting with the amino acid identified refers to attractive forces including covalent or non-covalent binding e.g. by chemical bonds, forming salt bridges by electrostatic interaction, hydrogen bond involving a hydrogen bond donor and acceptor or hydrophobic association e.g. via small or large van der Waals forces.

The molecules are determined and modelled to interact with at least one of the identified amino acids. These molecules should alter the activity of the FAS I complex influencing at least the enzymatic reaction rate of one of the enzymes present in the FAS I complex accordingly. For example, these molecules are candidate molecules for inhibitors of an enzymic activity or allow to increase yield or increase selectivity in the production of fatty acids by the FAS I complex.

The skilled person is well aware of suitable tools allowing the same. These tools include software like Autodock, AutoDock Vina, Dock, FlexAlD, LeDock, Glide, Molecular Operating Environment (MOE), rDock, SEED, SwissDock, CSD Gold, Chimera, DockThor, etc.

In a further aspect of the *in silico* method, an *in silico* method is provided of identifying molecules capable or of selectively altering the activity of an enzyme domain present in the FAS whereby the molecules determined and modeled show interaction under physiological conditions with at least one amino acid defined. As outlined above, suitable tools are available for doing so. The molecules determined and modeled may interact with amino acids present in the catalytic site of the respective enzyme or, alternatively or additionally, interact with adjacent regions of the same enzyme or a different enzyme present in the FAS I complex. These molecules may allow to selectively increase the production of specific fatty acid molecules, e. g. short fatty acid molecules or long fatty acid molecules as defined herein.

In a further aspect, a method of identifying a compound capable of altering at least one of the enzymatic activities of FAS I complex is provided. Said method comprises bringing into contact a candidate compound with a protein or polypeptide according to the present invention or with the FAS I complex in general and determining whether said candidate compound may interact with at least one amino acid of the amino acids in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7. In addition or alternatively it is determined whether said candidate compound is a competitor of ACP in interacting with at least one amino acid with the at least one amino acid in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131 of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7.

The method may include determining the altered activity by LC/MS methods known to the skilled person and described in the examples. This method is particularly suitable for high throughput screening of compound libraries. The candidate compounds may represent compounds starting as starting point for the development or suitable active agents. Said active agents may be fungicides or actinobacterial biocides. Typically, the step of bringing into contact the candidate compound with the FAS I complex is done in cell culture or *in silico.* Suitable cells involved in this method include host cells as described herein expressing the FAS I complex wherein the protein or polypeptide according to the present invention may be present or absent. That is, the FAS I complex present in the host cell may be a mutated one or may be a natural one.

In a further aspect, the present invention relates to a protein having mutations of at least one amino acid at a position corresponding to 5-11, 20-25, 34-105, 135-147 of SEQ ID No. 7 or not containing the amino acid sequence of 1 to 79 of SEQ ID No. 7.

That is, in a further aspect the present invention relates to altered ACP polypeptides or proteins wherein at least one amino acid in a position mentioned is mutated. Said mutation may be any kind of mutation as described herein. In a preferred embodiment, the mutations are at least one amino acid substitution in the ACP domain at a position corresponding to 40-49, 51-70, 72-105 of SEQ ID No. 7 or consisting of amino acids 80-169 of SEQ ID No. 7 or to functionalized fragments thereof. The respective proteins with the mutations mentioned above, are particularly suitable as competitors of ASP binding in the FAS I complex. In particular, it is submitted that the ACP polypeptides or proteins containing at least one of the above-mentioned substitutions or at least one amino acid substitution of SEQ ID No. 7 as shown in table 1 selectively alter the activity of the FAS I complex by changing the enzymatic rate accordingly.

In another aspect of the present invention, the protein with the mutations in SEQ ID No. 7 is particularly useful as a fungicide or actinobacterial biocide.

Said proteins e.g. for use as a fungicide or actinobacterial biocide may be provided in suitable forms for pharmaceutical compositions or agriculture compositions. The skilled person is well aware of providing suitable compositions accordingly.

### Brief description of the figures

**Figure 1****.** Structure of the S. cerevisiae FAS at 1.9 Å resolution.
   (A) The yeast fatty acid biosynthesis cycle is depicted; ACP: Acyl carrier protein, AT: Acetyltransferase, MPT: Malonyl-palmitoyl-transferase, KS: Ketosynthase, KR: Ketoreductase, DH: Dehydratase, ER: Enoyl reductase, FMN: Flavin mononucleotide.
   (B) Visualized chemical details in the 1.9Å structure. One of the six asymmetric units of FAS (top left) is extracted and depicted in the center. Individual enzymatic domains are shown following the same scheme as in (A). The locations of zooms (i-v) in the asymmetric unit are indicated. (i) FMN bound in the ER active site. The density indicates a 22° kink in the isoalloxazine ring system, consistent its reductive role in FA biosynthesis. (ii) FMN recognition by ER active site amino acids and their accompanying water network. (iii) NADP+ bound in the ER active site cleft with its nicotinamide group stacked between the FMN isoalloxazine and His740 corresponding to aa 184 in SEQ ID No. 1, a solvent network aids in recognition of the ADP moiety. (iv) Recognition of NADP+ by amino acids and solvent in the KR active site cavity. (v) Malonyl-CoA was found in a post hydrolysis state in the MPT active site. CoA interacts with many MPT active sites residues, while the malonyl group is covalently attached to Ser1808 corresponding to aa 244 in SEQ ID No.6 interacting with Arg1834 corresponding to aa2 70 in SEQ ID No.6.

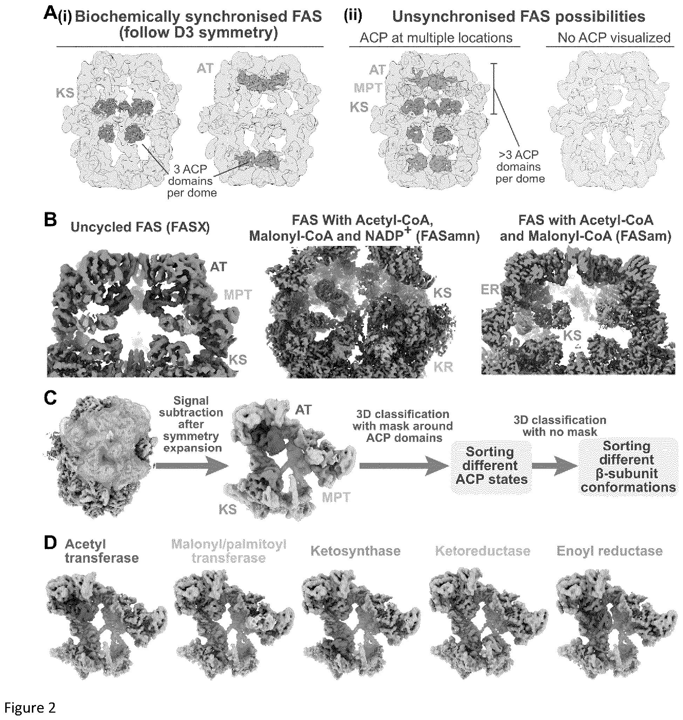

**Figure 2****.** Visualizing ACP domains in positions representing intermediates in fatty acid synthesis.
   (A)(i) Only three unique ACP densities are visualized in each dome of biochemically synchronized FAS. (ii) Two scenarios are conceivable in unsynchronized FAS: Either ACP domains remain unresolved (right), or densities greater than three are visualized (left). Both scenarios would imply asynchronous binding of the three ACP domains to different enzymatic domains within a FAS dome.
   (B) Distribution of ACP domains in uncycled FAS (FASx, left), FAS in the presence of Acetyl-CoA, Malonyl-CoA, and NADP+ (FASamn, middle), and FAS in the presence of Acetyl-CoA and Malonyl-CoA (FASam, right).
   (C) Image processing scheme for sorting different ACP bound states.
   (D) Structures of FAS asymmetric units with ACP visualized in positions representing five intermediates in FA biosynthesis as indicated.

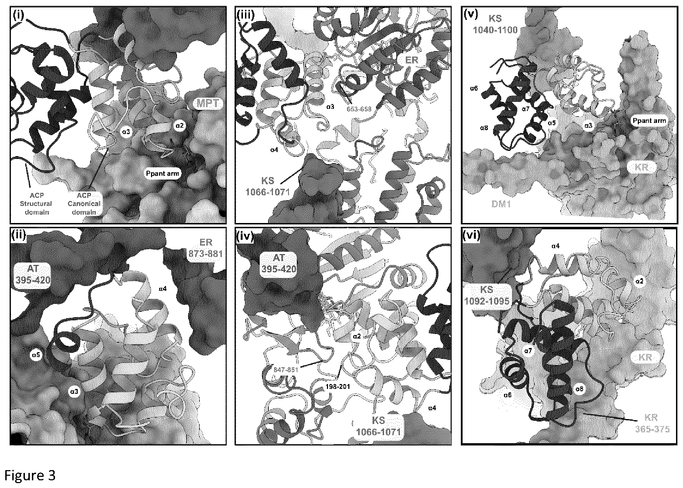

**Figure 3****.** ACP FAS enzymatic domain interactions.
   Interactions of the ACP when bound to MPT **(i,ii),** ER **(iii,iv),** and KR domains **(v,vi).** ACP helices α2, and α3 contact residues outside the MPT active site cleft **(i),** whereas helix α4, and the loop between helix α4/5 contact structural segments of the ER (874-880 corresponding to aa 318-324 in SEQ ID No.1) and AT (402-406 corresponding to aa 402-406 in SEQ ID No.2) domain, respectively **(ii).** At the ER domain, ACP helix α3 contacts the ER loop (653-658 corresponding to aa 97-102 in SEQ ID No.1) whereas helix α4 lies adjacent to the structural segments (1066-1071 corresponding to aa 96-101 in SEQ ID No.3) of the KS domain **(iii).** Further, ACP helix α2 contacts the adjacent AT segment (402-407 corresponding to aa 402-407 in SEQ ID No.2) whereas ACP residues (198-201 corresponding to aa 62-65 in SEQ ID No.7) are proximal to an ER loop (residues 847-851 corresponding to aa 291-295 in SEQ ID No.1) **(iv).** At the KR domain, both canonical and structural ACP domains are involved in binding. ACP helices α2 and α3 contact residues near the KR active site **(v),** whereas the canonical domain rests upon the DM1 helix contacting residues 365-375 corresponding to aa 38-48 in SEQ ID No. 5 **(vi).** The ACP loop between helix α4/5 also forms contacts with the adjacent KS residues 1092-1095 corresponding to aa 122-125 in SEQ ID No. 3 **(vi).**

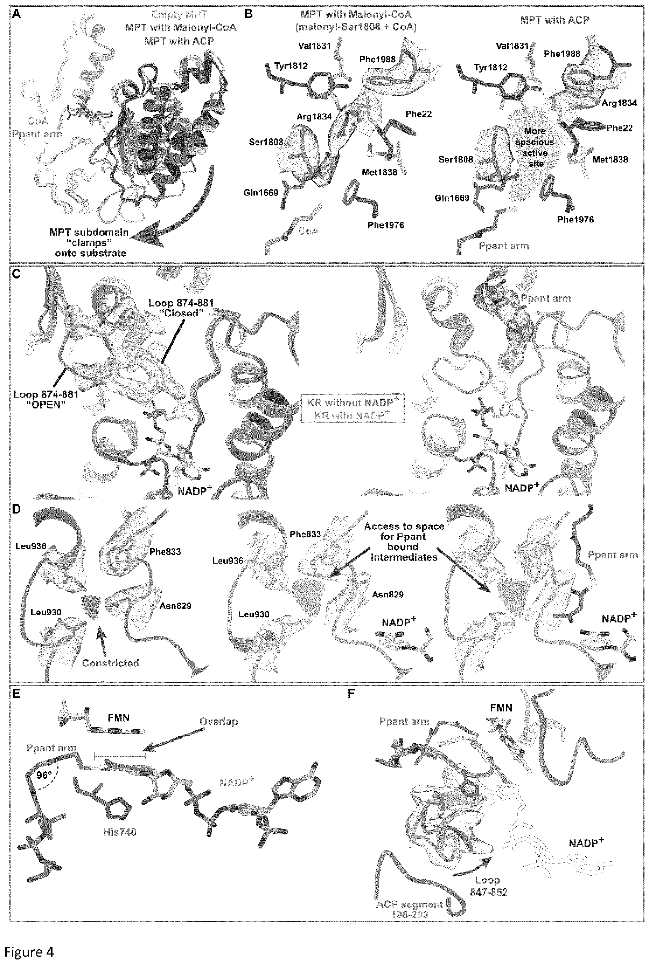

**Figure 4****.** Structural rearrangements upon substrate- and ACP binding.
   (A) Overlay of empty, malonyl-CoA bound, and ACP bound MPT domains.
   (B) MPT active site comparison in the presence of malonyl-CoA (left) and in the ACP bound state (right).
   (C) Overlay of the KR active site in the presence and absence of NADP+.
   (D) KR active site in the absence of NADP+ (left), with bound NADP+ (middle), and NADP+ and ACP together (right).
   (E) Overlay of Ppant-arm and NADP+ at the ER, depicting the mutually exclusive binding of these two entities as a consequence of a steric clash.
   (F) Overlay of the ER active site with bound NADP+ and bound ACP. The loop 847-852 corresponding to aa 291-296 in SEQ ID No.1 is shifted inwards when ACP is bound resulting in a narrower ER active site cleft.

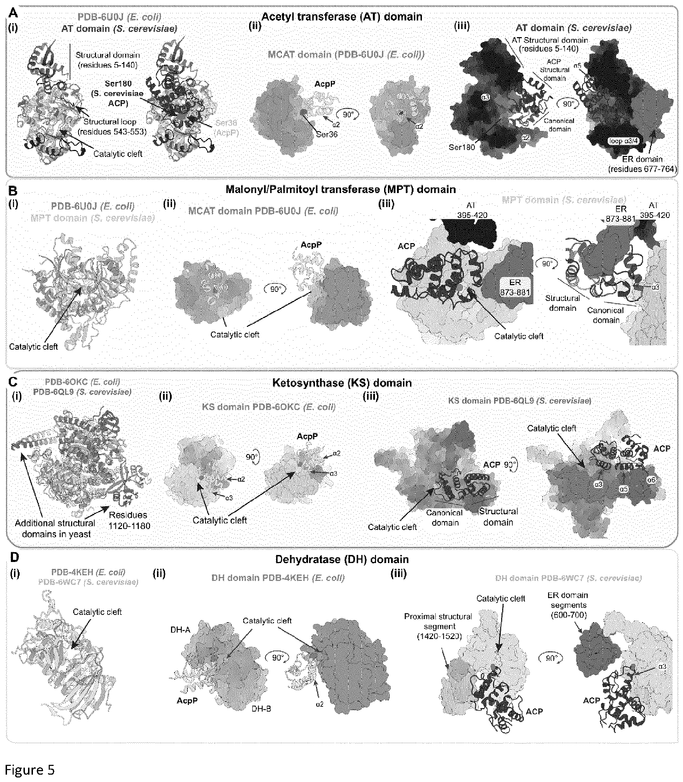

**Figure 5****.** ACP binding and domain comparison between bacterial type II and S. cerevisiae type I FAS systems, related to Figures 3 and 4.
   The ACP bound malonyl coenzyme A- acyl carrier protein transacylase (MCAT) enzyme (E. coli, PDB ID: 6U0J), which is homologous to S. cerevisiae type I FAS AT and MPT domains and the ACP bound structures of E. coli dehydratase (FabA, PDB ID: 4KEH6), Ketosynthase (FabB, PDB ID: 6OKC) were compared to the fungal counterparts.
   (A) Acetyl transferase domain.
      (i, left) Overlay of E. coli MCAT enzyme with the scFAS AT domain. The scFAS AT domain has structural segments in addition to the conserved fold of the catalytic domain. (i, right) Overlay of ACP domain bound to the E. coli MCAT enzyme (AcpP) and scFAS AT domain (ACP).
      (ii and iii) Comparison of ACP bound to the E. coli MCAT enzyme (ii) with ACP bound to scFAS AT domain (iii). Even though the Ppant-Serine (Ser36 for E. coli AcpS and Ser180 corresponding to aa 44 in SEQID No7, of scFAS ACP) are located at the entrance of the catalytic cleft in both ACP bound structures, the interaction interfaces between ACP, and catalytic domains differ. The scFAS ACP cannot bind in the AcpP-MCAT pose due to clashes with the AT structural loop (543-553 corresponding to aa 543-553 in SEQ ID No.2), and the adjacent ER domain segments (ii, iii). Instead, scFAS ACP interacts with the AT catalytic domain via α-helices 2 and 3, with ER domain segments using the loop connecting α-helices 3 and 4, and via helix 5 to the AT structural domain (iii). The scFAS AT-ACP interaction interface with a buried surface area of ~1033 Å2 is almost twice as large as the MCAT-AcpP interaction which buries a surface of 459 Å2.
   (B) Malonyl/Palmitoyl transferase.
      (i) Overlay of E. coli MCAT enzyme with the scFAS MPT domain. The scFAS MPT domain also has structural segments in addition to the conserved fold of the catalytic domain.
      (ii and iii) Comparison of AcpP bound to E. coli MCAT enzyme (ii), with ACP bound to scFAS MPT domain (iii). The position of the Ppant-Serine is indicated. AcpP contacts MCAT via its α-helix 2 and the loop connecting α-helices 2 and 3. In comparison, scFAS ACP contacts the MPT domain via its α-helix 3 and nearby structural segments of ER, and AT domains through α-helices 4 and 5, respectively. These additional interactions stabilize the ACP
   (C) Ketosynthase.
      (i) Overlay of E. coli KS enzyme with the scFAS KS domain. Similar to bacterial KS, the scFAS KS domain dimerises, however, in addition to the conserved catalytic domain, structural segments exist in scFAS KS.
      (ii and iii) Comparison of ACP bound to E. coli KS enzyme (AcpP) (ii) with ACP bound to scFAS KS domain (ACP) (iii). The position of the Ppant-Serine is indicated. AcpP contacts the KS via α-helices 2 and 3, whereas in scFAS ACP α-helix 3 of the canonical domain contacts the KS domain in a manner distinct to the AcpP-KS interaction. The scFAS ACP-KS interaction is additionally stabilized by ACP α-helices 5 and 6 of the structural domain interacting with KS structural segments (1120-1180 corresponding to aa 150-210 in SEQID No.3).
   (D) Dehydratase.
      (i) Overlay of E. coli dehydratase enzyme with the scFAS dehydratase domain12. The E. coli dehydratase (FabA) forms a dimer, whereas in scFAS it is has a pseudo-dimeric organisation.
      (ii and iii) Comparison of ACP bound to E. coli DH enzyme (AcpP) (ii), with ACP bound to scFAS DH domain (ACP) (iii). The position of the Ppant-Serine is indicated. E. coli AcpP interacts with FabA mainly via its α-helix 2. The interaction of ACP with scFAS DH is primarily mediated by α-helix 3, and the poses of the bacterial and scFAS ACPs differ with respect to the respective DH. In the case of scFAS ACP, proximal DH structural elements (1420-1520 corresponding to aa 179-279 in SEQ ID No.4) and the ER domain prevent adopting an AcpP-like binding pose (iii).

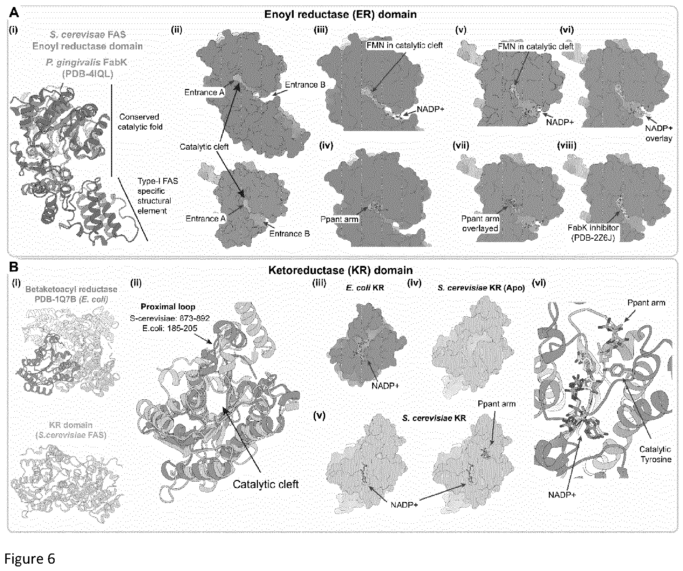

**Figure 6****.** Comparison of structural features of bacterial type II Enoyl- and Ketoreductases to fungal type I counterparts, related to Figures 3 and 4.
   Since, there are presently no deposited structures of ACP bound to the fungal like ER (FabK) and KR domains of the type II FAS system, only broad comparisons can be performed with regards to complementarity of ACP recognition between type I and type II systems in these cases.
   (A) Enoyl reductase.
      (i) Overlay of P. gingivalis FabK enzyme (PDB ID: 4IQL) with the scFAS ER domain.
      (ii) Surface representation of FabK and scFAS ER domain. Both scFAS ER domain and the bacterial FabK exhibit an active site cleft with two entrances and a bound FMN cofactor, with additional segments in scFAS ER playing a structural role.
      (iii and iv) NADPH/NADP+ enters through entrance B (iii), whereas the ACP-bound Ppant-group gains access through entrance A (iv).
      (v and vi) In the FabK-NADP+ structure, only the ADP moiety was resolved (v), overlay of the NADP+-bound scFAS ER with FabK (vi) suggests that the NADPH/NADP+ would be bound in a similar manner by FabK.
      (vii and viii) Overlaying Ppant-group-bound scFAS ER with FabK (vii), suggests that entrance A in FabK would be able to similarily accommodate a Ppant-group into the active site. Notably, a FabK inhibitor binds in the Ppant binding pocket and acts as a competitive inhibitor to NADPH/NADP+ (viii).
   (B) Ketoreductase.
      (i) Bacterial KR (FabG, PDB ID: 1Q7B) is a tetramer, whereas the scFAS KR dimerises.
      (ii, iii, iv) The catalytic fold of the KR is well conserved between the two FAS system types. The active site cleft NADP+-bound E. coli KR (iii) is much more open than the NADP+-bound scFAS KR (v, left), where the proximal loop clamps onto the bound co-substrate and creates a pocket for Ppant-arm entry (v, right).
      (vi) Overlay of the NADP+-bound FabG with ACP-bound scFAS KR domain. As both bound NADP+ and the catalytic tyrosine residue adopt similar positions in both type II and type I KR, one can infer that the Ppant-arm in bacterial KR would be similarly positioned as observed here for scFAS KR.

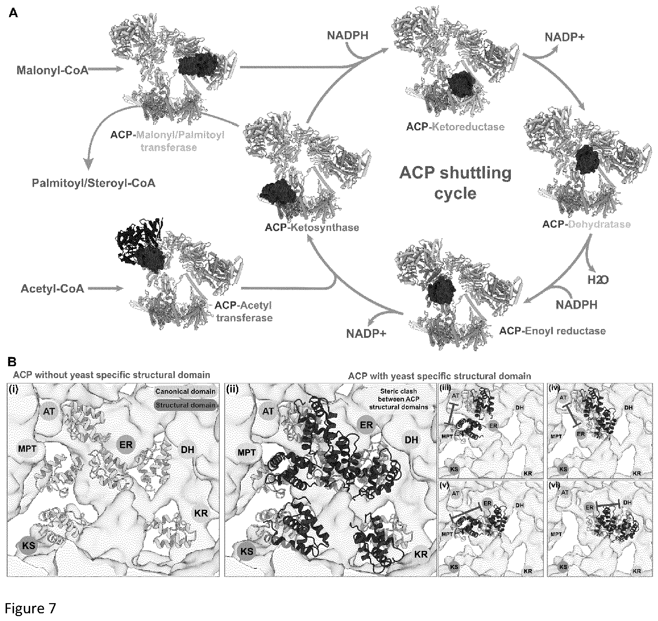

**Figure 7****.** Structural snapshots of ACP domains in the FA biosynthesis cycle.
   (A) Architectural framework of the yeast FA biosynthesis cycle. Shown are asymmetric units of yFAS and ACP domains occupying distinct positions, representing intermediates in the FA biosynthesis cycle. In these distinct positions, the domain to which ACP is bound is colored: AT, MPT, KS, KR, DH, and ER. All states were resolved herein with exception of the previously reported DH bound state.
   (B) Comparison of ACP domain binding to enzymatic domains in the absence (i) and presence (ii) of the yeast-specific structural ACP domain. When bound to AT (iii, iv), MPT (iii, v), ER (iv, v, vi), and DH domains (vi), the structural domain exerts a steric hindrance to ACP binding to the adjacent enzymatic domain. In the absence of the ACP structural domain such binding is unimpeded. A "path of least resistance" is thus established that delineates a `functional compartment' where active sites are arranged as depicted in (ii).

The present invention will be described further by way of examples without limiting the same.

### Material and Methods:

### DATA AND CODE AVAILABILITY

The coordinates for the determined structures are deposited in the Protein Data Bank (PDB) accession codes: PDB: 8PRV, PDB: 8PRW, PDB: 8PS1, PDB: 8PS2, PDB: 8PS8, PDB: 8PS9, PDB: 8PSA, PDB: 8PSF, PDB: 8PSG, PDB: 8PSJ, PDB: 8PSK, PDB: 8PSL, PDB: 8PSM. EM maps are deposited with the (EMDB) accession codes: EMDB: 17839, EMDB: 17840, EMDB: 17842, EMDB: 17843, EMDB: 17846, EMDB: 17847, EMDB: 17848, EMDB: 17851, EMDB: 17852, EMDB: 17853, EMDB: 17854, EMDB: 17855, EMDB: 17856, EMDB: 17859.

### EXPERIMENTAL MODEL AND STUDY PARTICIPANT DETAILS

### Yeast culture

Saccharomyces cerevisiae strain tma17Δ BJ2168 (MATa prc1-407 prb1-1122 pep4-3 leu2 trp1 ura3-52 gal2 tma17::kanMX), Singh, K., et.al., (2020), see above, was used herein. Cells were grown in YPD medium in an Infors 250 litre fermenter and harvested in late log phase at an OD600 of 9-10. Subsequently, cells were washed with cold ddH2O and resuspended in 2X purification buffer (0.05 M BisTris pH 6.5, 0.05 M potassium acetate, 0.01 M magnesium acetate) containing 20% (w/v) sucrose such that 2 mL of buffer was added per gram of cells. Cells were then flash frozen as beads in liquid nitrogen and stored at -80°C until further use.

### Bacterial culture

For expression of γ-1xUnaG, γ-2xUnaG, γ-Tes, or Tes the respective plasmids were transformed into chemically competent BL21-AI (ThermoFisher Scientific; γ-Tes or Tes) or BL21 Star (ThermoFisher Scientific; γ-1xUnaG or γ-2xUnaG) cells. Starting cultures were grown in LB with 1% (w/v) glucose over night at 37°C. 500 mL cultures in TB medium inoculated using the pre- culture to an OD600 of 0.05 were grown with 1% glucose (w/v) and antibiotics. Cultures were grown (37°C, 180 rpm) until an OD600 of 0.6-0.8 was reached, the temperature lowered to 18°C, and after 1 hour incubation cultures were induced by addition of 1 mM IPTG (BL21 Star cultures), or 1 mM IPTG and 2% (w/v) L-arabinose (BL21-AI cultures). Overexpression was performed for 16 hours at 18°C. Cells were harvested by centrifugation (5,000 g, 5 min, 4°C), resuspended and washed with ice-cold 1x PBS-buffer (137 mM NaCl, 2.7 mM KCI, 10 mM Na2HPO4, 1.8 mM KH2PO4; pH 7.4), and centrifuged again (5000 g, 5 min, 4°C). The final pellet was flash frozen in liquid nitrogen and stored at -80°C.

### Purification of yeast FAS

FAS was purified as described by Singh, K., et.al., (2020), see above. It was started with 700 g of frozen cell beads (corresponding to 233 g wet cell weight of yeast), which were ground in liquid nitrogen to a fine powder using a Retsch ZM200 mill. The ground powder was thawed in a water bath at 37°C, supplemented with purification buffer (0.05 M BisTris pH 6.5, 0.05 M potassium acetate, 0.01 M magnesium acetate) to 0.33X concentration from a 10X stock, followed by the addition of sucrose powder to 20% (w/v), benzamidine chloride to 10 mM and PMSF to 1 mM (from a 100 mM stock solution in propanol). The extract was incubated at 25°C on a magnetic stirrer for 30 minutes followed by centrifugation at 30,000 rcf for 30 minutes at 4°C. After centrifugation, the supernatant was filtered through 3 layers each of cheese cloth and miracloth to obtain a S30 yeast cell extract. To this, Octyl Glucose Neopentyl Glycol (OGNG) (from a 10% (w/v) stock) was added to a final concentration of 0.2% (v/v) and the extract was incubated at 30°C for 30 minutes followed by centrifugation at 100,000 rcf for 1 hour at 4°C. The supernatant was again filtered through 3 layers each of cheese cloth and miracloth. The thus clarified S100 extract was subjected to differential precipitation with PolyEthyleneGlycol400 (PEG; number signifies the mean molecular weight of the PEG polymer). PEG400 was added to a concentration of 20% (v/v) to the yeast S100 extract while stirring at 18°C and incubated for 30 minutes. Precipitated proteins were removed by centrifugation at 30,000 rcf for 30 minutes at 4°C. The supernatant was then precipitated by raising the concentration of PEG400 to 30% (v/v) as described above. The precipitate of this step, which contains FAS, was recovered by centrifugation at 30,000 rcf for 30 minutes at 4°C and resuspended in purification buffer containing 2% (w/v) sucrose, 10 mM DTT and 0.01% (w/v) Lauryl Maltose Neopentyl Glycol (LMNG) in an orbital shaker at 18°C. The resuspended material was loaded on 10-45% (w/v) linear sucrose gradients in purification buffer containing 10 mM DTT, which were centrifuged at 100,000 rcf for 16 hours at 4°C. Gradients were harvested in 1 mL fractions. SDS-PAGE was utilized to identify fractions containing FAS. Selected fractions were pooled and precipitated by the addition of 40% (v/v) PEG400. After centrifugation (30,000 rcf, 30 minutes), the supernatant was removed and the precipitate was resuspended in purification buffer containing 2% (w/v) sucrose, 10 mM DTT and 0.01% (w/v) LMNG. The resuspended material was loaded on linear 10-45% (w/v) sucrose gradients in purification buffer containing 10 mM DTT, and centrifuged at 79,000 rcf for 16 h at 4°C. Fractions containing FAS were identified by SDS-PAGE, pooled and cycled in the presence of 50 µM Malonyl CoA and 100 µM of NADPH for 30 min at 18°C ("cycled" FAS). For the FASx sample, cycling of FAS by addition of Malonyl CoA and NADPH was omitted ("uncycled" FAS). The protein was precipitated and concentrated by the addition of 40% (v/v) PEG400 and resuspended in purification buffer containing 2% (w/v) sucrose, 10 mM DTT and 0.01% (w/v) LMNG. Another round of linear 10-45% (w/v) sucrose gradients in purification buffer containing 10 mM DTT, centrifuged at 60,000 rcf for 16 hours at 4°C and subsequent re-precipitation of FAS fractions with 40% (v/v) PEG400 was required to yield a final purified protein preparation at ~15 mg/mL in purification buffer containing 10% sucrose (w/v), 10 mM DTT and 0.01% (w/v) LMNG. Protein concentrations were determined by Bradford assay (BioRad, Munich, Germany) using a BSA standard. The protein concentration was kept at ~15 mg/mL.

### Purification of recombinant proteins

For the purification of γ-1xUnaG, γ-2xUnaG, γ-Tes, or Tes the frozen cell paste from overexpressing cells were resuspended in 5 mL resuspension buffer per gram of cells. The suspension was incubated (30 min, 4°C, mixing) after adding 2 U/mL DNAse (NEB) and 0.33 mg/mL lysozyme. Cells were lysed by passing two rounds through an Emulsiflex C3 (Avestin) at 15,000 PSI, the lysate was centrifuged (50,000 g, 30 min, 4°C) filtered through a cellulose-acetate syringe filter (Sartorius Stedim) and loaded on a 10 mL Ni-NTA column pre-equilibrated in 20 CV of resuspension buffer (0.05 M BisTris pH 6.5, 0.05 M potassium acetate, 0.01 M magnesium acetate, 0.01 M Imidazole). After binding of the protein of interest, the column was washed with 10 CV of washing buffer (0.05 M BisTris pH 6.5, 0.5 M potassium acetate, 0.01 M magnesium acetate, 10 mM Imidazole). For elution, 5 CV of elution buffer (0.05 M BisTris pH 6.5, 0.05 M potassium acetate, 0.01 M magnesium acetate, 0.5 M Imidazole) was applied and 1 mL fractions collected. The elution fractions were analyzed by SDS-PAGE and corresponding fractions pooled. The pooled protein solution was then digested by addition of TEV-protease in an enzyme-to-protein ration of 1:50. The digestion reaction was performed overnight (16 hours, 4°C, gently mixing) in a dialysis tube (6,000-8,000 MWCO) against 5 L of dialysis buffer (0.05 M BisTris pH 6.5, 0.05 M potassium acetate, 0.01 M magnesium acetate, 5% (w/v) sucrose). To remove the affinity tag, TEV protease, as well as undigested protein, the dialyzed protein solution was loaded on a 10 mL Ni-NTA column equilibrated with 10 CV dialysis buffer. The flow-through was collected and analyzed by SDS-PAGE. For concentration of the final protein, centrifugal filters of appropriate pore size were used, and the protein concentration was determined spectroscopically using the specific extinction coefficient as well as the molecular weight calculated from sequence. The final protein solutions were aliquoted, frozen in liquid nitrogen and stored at -80°C.

### Cryo-EM sample preparation

For the FASx sample, "uncycled" FAS was diluted to 0.5mg/mL using cryo buffer (0.05 M BisTris pH 6.5, 0.05 M potassium acetate, 0.01 M magnesium acetate, 10 mM DTT). For the 1.9 Å FAS structure (FASmn), cycled FAS was diluted to 2 mg/mL using cryo buffer and incubated with 1 mM Malonyl CoA and 2 mM NADP+ at 30°C for 30 min followed by transfer to 4°C. For the FASamn sample, cycled FAS was diluted to 2 mg/mL using cryo buffer and incubated with 20 µM Acetyl CoA, 1 mM Malonyl CoA and 2 mM NADP+ at 30°C for 30 min followed by transfer to 4°C. For the FASam sample, cycled FAS was diluted to 2 mg/mL using cryo buffer and incubated with 1 mM Acetyl CoA and 1 mM Malonyl CoA at 30°C for 30 min followed by transfer to 4°C. The sample were kept at 4°C until grid preparation.

### Cryo-EM grid preparation

UltrAuFoil R1.5/1.6 300-mesh grids (Quantifoil, Jena) were used for the FAS in the presence of Malonyl-CoA and NADP+. The grids were pre-floated with custom-made continuous carbon foil covering ~25% of the grid area for alignment purposes. For the FASx and FASam samples, Quantifoil R3.5/1 (Cu), 200 Mesh grids were used, whereas for the FASamn sample, Quantifoil R2/2 (Cu), 200 Mesh grids were used. The grids were glow discharged for 30 seconds with a glow discharger built in-house under low vacuum shortly prior to sample application. For the FASmn and FASamn samples, 4 µL of the respective protein solution was applied to the glow-discharged grids, which were blotted for 6.5 seconds and plunge-frozen with a Vitrobot Mark IV (Thermo Fisher Scientific, Eindhoven) operated at 4°C and 100% humidity. For the FASx and FASam sample, the particles were adsorbed to a continuous carbon film attached to a Quantifoil (3.5/1) (Quantifoil, Jena, Germany) grid for 2 min at 4°C. The grid was then transferred to a Vitrobot Mark IV plunge-freezer where 4 µL of ddH2O was applied to the grid followed by blotting and vitrification as described above.

### Cryo-EM data collection and image processing

### FAS structure in the presence of NADP+ and Malonyl-CoA

All cryo-EM data were collected in nanoProbe mode on a Titan Krios electron microscope operating at 300 kV equipped with a monochromator (ThermoFisher Scientific, Eindhoven) and an aplanatic image corrector "B-Cor" (CEOS, Heidelberg). The monochromator was tuned to operate at 3 kV potential and excitation (0.8) to obtain an energy spread of the source of about 0.1-0.15 eV. The B-Cor was set-up to correct for off-axial aberrations, and beam-image shift induced coma and higher order electron optical aberrations and for linear distortions. For each cryo-EM dataset, the B-Cor was tuned with the signals from the amorphous carbon i) to correct the phase errors introduced by on-axis electron optical aberrations to less than 45 degrees at scattering angles ≥ 17 mrad, which is equivalent to ≤1.16 Å resolution, and ii) to reduce linear distortions to ≤0.2%. A total of 30659 movies with 40 fractions each were collected (Table 2). The exposure time was over 18.54 seconds with a dose of ~1.25 e/Å2 per fraction in electron counting mode using a Falcon III direct electron detector (ThermoFisher Scientific, Eindhoven) at a nominal magnification of 96000X (~0.615 Å/pixel) and 0.5 µm to 2.4 µm underfocus resulting in a total dose of ~50 e/Å2. An adapted version of the software EPU (Thermo Fisher Scientific, Eindhoven) was used to acquire data by beam-image shift over up to 3 X 3 holes where five movies were acquired per hole. All image processing was performed using RELION 3.1 Zivanov, J., et.al., (2020), IUCrJ 7, 253-267. 10.1107/S2052252520000081, if not indicated otherwise. Global motion correction and dose-weighting were performed using RELION's implementation of MotionCorr2, Zheng, S.Q., et. al., (2017). Nat. Methods 14, 331-332. 10.1038/nmeth.4193 with a B-factor of 150. The power spectrum of aligned frames were used for CTF estimation by CTFFIND 4.1.13, Rohou, A., and Grigorieff, N. (2015). J. Struct. Biol. 192, 216-221. 10.1016/j.jsb.2015.08.008. Particles were picked with reference to 2D templates using Gautomatch, a freely available software, and extracted with 4X binning (4.26 Å/pixel, 90 pixel box). Three rounds of reference-free 2D classifications were performed to remove bad particles. The remaining particles were re-centered and re-extracted in a 180 pixel box (2.13 Å/pixel) and subjected to 3D classification using C1 symmetry to distinguish intact particles from broken ones. The FAS structure (EMDB-4578) was low pass filtered to 45 Å and used as the 3D reference. The intact particles were then selected and re-extracted in a 560 pixel box (0.615 Å/pixel). 3D refinement while imposing D3 symmetry was then performed, resulting in a 2.45 Å structure (FSC 0.143 criterion). A round of CTF refinement was performed on the 3D refined particles to correct for per-micrograph defocus and per-micrograph astigmatism. Then, particle motion and the corresponding per-frame relative B factors were estimated by Bayesian polishing. The polished particles were subjected to a second round of 3D refinement which resulted in a 1.98 Å structure. Another round of CTF refinement was performed to correct for per-particle defocus and per-particle astigmatism. Afterwards, the particles were again subjected to Bayesian polishing where they were extracted with 920-pixel box (0.615 Å/pixel) followed by a third round of 3D refinement. Ewald sphere correction, Zivanov, J., et.al., (2018), Elife 7. 10.7554/eLife.42166, and Russo, C.J., and Henderson, R. (2018), Ultramicroscopy 187, 26-33. 10.1016/J.ULTRAMIC.2017.11.001 was performed on the two resulting half-maps yielding a final reconstruction with a nominal resolution of 1.87 Å.

### FAS asymmetric units in the FASx, FASamn and FASam samples.

Data were acquired on a Titan Krios (ThermoFisher Scientific) operating at 300 kV using a Falcon 3 (linear mode) detector. Data collection and processing statistics are summarized in Table 2. Image processing of the acquired movies were performed in a similar manner as for described above until the 3D classification step. The selected intact particles were then used for another round of 3D classification (C1 symmetry).

In the FASx dataset, 162079 particles adopted a rotated conformation whereas 35927 particles were in the non-rotated conformation. The two sets of particles were processed separately. The selected particles were then used for 3D refinement (C1 symmetry). In particles with a rotated conformation, three ACP densities were found per dome adjacent to the AT domains. In the particles with a non-rotated conformation, nine ACP densities were found per dome; three each adjacent to the AT, KS and MPT domains. Since, each FAS dome can contain only 3 ACP domains, the particles with the non-rotated conformation were used for further rounds of 3D classification (C1 symmetry). However, all the 3D classes of FAS obtained contained at least 6 partial ACP densities per dome suggesting a random distribution of the ACP domains within the two domes. To sort for the different ACP bound states, a strategy involving symmetry expansion and signal subtraction was used in a similar manner as described before, Roh, S.H., et.al., (2017), Proc. Natl. Acad. Sci. U. S. A. 114, 8259-8264. 10.1073/pnas.1704725114. For this, the particle belonging to the non-rotated conformation were used for 3D refinement (D3 symmetry) followed by symmetry expansion using "relion_particle_symmetry_expand" function in RELION. A mask comprising of an αβ dimer as well as ACP domains at the AT, KS and MPT domains was used to in silico extract each asymmetric unit from each FAS molecule. The asymmetric unit were then 3D classified without alignment using a mask around the three possible positions of the ACP domain to sort for the different ACP's located at different active sites. Four distinct classes were observed where the ACP was located at either AT/KS/MPT domain or not visualized. The particles from each class were then 3D classified (without alignment) separately without any mask to sort for different conformational state of the αβ dimer. The particles with the rotated conformation were also processed in a similar manner as for the ones adopting the non-rotated conformation.

In the FASamn dataset, all particles were found to have a non-rotated conformation after 3D classification. However, each dome contained six ACP densities, three for ACP at the KR domain and three for ACP at the KS domain. Therefore, particle symmetry expansion and signal subtraction followed by 3D classification as described for the FASx dataset was performed.

In the FASam dataset, all particles were found to have a non-rotated conformation after 3D classification. However, each dome contained six ACP densities, three for ACP at the ER domain and three for ACP at the KS domain. Therefore, particle symmetry expansion and signal subtraction followed by 3D classification as described for the FASx dataset was performed.

### Cryo-EM Model building

The crystallographic Δ γ-FAS model (PDB-6QL9) was used as the initial model for the 1.87Å FAS map. It was docked into the EM densities as a rigid body using UCSF Chimera, Pettersen, E.F., et.al., (2004), J. Comput. Chem. 25, 1605-1612. 10.1002/jcc.20084. An additional round of rigid body refinement was then performed in Refmac5, Murshudov, et.al., (2011), Acta Crystallogr. D. Biol. Crystallogr. 67, 355-367. 10.1107/S0907444911001314. The model then underwent several rounds of manual modelling in Coot33 and refinement in Refmac5. Water molecules were built into the map through a combination of Coot and manual modelling and all waters with a Bfactor above 70Å2 after refinement were not included.

The model obtained from the 1.87 Å FAS map was then used as the initial model for the FAS asymmetric units with ACP domains bound to enzymatic domains. The asymmetric unit comprising a single αβ dimer was docked into the EM densities as a rigid body using UCSF Chimera and then the models underwent several rounds of manual modelling in Coot, Emsley, P., and Cowtan, K. (2004), Acta Crystallogr. Sect. D Biol. Crystallogr. 60, 2126-2132. 10.1107/S0907444904019158 and refinement in Refmac5.

For model validation, Fourier Shell Correlation (FSC) between the refined model and the map was calculated (FSCsum). The model was also refined using the first unfiltered half-map. This model was then compared to the first half map (FSCwork) as well as to the second half-map (FSCfree) to check for signs of overfitting. In table 2, the RMS deviation are from Refmac5 whereas the other values were calculated using the Phenix software package, Adams, P.D., et- al., (2010), Acta Crystallogr. Sect. D Biol. Crystallogr. 66, 213-221. 10.1107/S0907444909052925. All figures were made using Pymol36, UCSF Chimera and ChimeraX, Goddard, T.D., et.al., (2018), Protein Sci. 27, 14-25. 10.1002/pro.3235.

### Enzyme assays and semiquantitative analysis of CoA derivatives

For semiquantitative product analysis measurements of different FAS enzyme assays, 253.8 nM of freshly purified FAS was mixed with a 500X molar excess of γ-subunit or TesA-constructs, respectively. All reactions were incubated for 30 min at 25°C to ensure a binding of the added proteins to FAS and subjected to density gradient centrifugation. To the density gradient peak fractions, final co-substrate concentrations of 100 µM (acetyl-CoA), 60 µM (malonyl-CoA) and 320 µM (NADPH) were added and enzymatic reactions initiated. After an incubation for 30 min at 30°C, the reactions were quenched by shock-freezing in liquid nitrogen. In the subsequent treatment with TesA 6 µM was added before shock-freezing. CoA thioesters were analyzed according to a method adapted from, Wolf, J., et. al., (2016), Mol. Microbiol. 102, 882-908. 10.1111/mmi.13498. Methanol was replaced by acetonitrile to facilitate the analysis of acyl-CoAs up to C20-acyl-CoA, and the gradient was adapted to allow the separation of derivatives of higher polarity (e.g. malonyl-CoA and free CoA). The metabolites were analyzed by HPLC-MS using an Agilent 6545 QTOF system equipped with an ESI source coupled to a 1290 series HPLC system. One µL of the enzyme assay without any further treatment was directly injected onto a Gemini C18 column (3 µm, 110 Å, 150 X 2 mm). The solvent used were 50 mM ammonium formate, pH 8.1 (A) and acetonitrile (B) at 35°C with the following gradient: 95% A for 1 min, gradient to 30% B in 18 min, gradient to 95% B in 17 min and gradient to 100% B in 4 min. The QTOF was run at following settings: positive mode, gas temperature 200°C, Sheath gas temperature 325°C, Fragmentor 180 V, Skimmer 45 V, Capillary voltage 4500 V. Data were evaluated using the MassHunter Qualitative Navigator software (version B08.00, Agilent Technologies, Santa Clara, CA, USA) by extracting ion chromatograms for the [M+H]+ and the [M+2H]++ m/z of the respective CoA thioesters. The retention time was confirmed by the use of authentic standards.

### Sequence conservation analysis

The inventors analysed the sequence conservation of ACP binding sites at different catalytic sites between S. cerevisiae and Y. lipolytica. For this, they first generated a multiple sequence alignment of the FAS genes from these two species using Clustal Omega, Madeira, F., et.al., (2022), Nucleic Acids Res. 50, W276-W279. 10.1093/nar/gkac240 followed by mapping the sequence conservation onto the different ACP bound states of S. cerevisiae FAS using ChimeraX.

### QUANTIFICATION AND STATISTICAL ANALYSIS

### Calculating ACP distribution per asymmetric unit

Based on the results of the 3D classification after signal subtraction a label was assigned to every asymmetric unit. Asymmetric units were then traced back to their respective FAS molecules in the RELION particle star file. The information gathered was then compiled into a table and analyzed in a similar manner as described by Roh, et.al., (2017), Proc. Natl. Acad. Sci. U. S. A. 114, 8259-8264. 10.1073/pnas.1704725114. In brief, we used computational analysis to capture correlation among asymmetric units by using normalized mutual information, which measures the conformational correlation between a pair of subunits. To compare the NMI distribution of the data with a random distribution, we generated 100 replicate shuffled datasets based on the original data. In the replicated data, each particle in the original data was rotated at random. The calculation of ACP distribution per asymmetric unit/FAS molecule was then performed using PANDAS, Reback, J., et al. (2020), pandas-dev/pandas: Pandas 1.0.3. 10.5281/ZENODO.3715232.

### Semiquantitative analysis of CoA derivates

All enzymatic reactions were performed in triplicate. The total CoA content was expressed as 100% for each individual reaction. From these values averages and error bars calculated. The relative abundance of individual acyl-CoAs is represented as a fraction of the total CoA content.

### Results

### Structure of yeast FAS at better than 2Å resolution

To reduce potential structural heterogeneity by employing FAS purified from a S. cerevisiae strain with a chromosomally deleted gamma-subunit (Δγ-FAS) has been used herein, Singh, K., et.al., (2020). Δγ-FAS was additionally conformationally stabilized by incubation with saturating concentrations of malonyl-CoA and NADP+ prior to cryo-EM grid preparation. EM grids were imaged using a 300kV Titan Krios Mono/BCOR microscope, resulting in a dataset with an experimental B-factor of ~53 Å2, and yielding a structure at a nominal resolution of 1.9Å. The local resolution of the map varied from 1.8-2.2Å at the central wheel, to 2.2-2.7Å at the dome region. This spread in local resolution has been observed earlier. Notably, optical improvements in the EM proportionally allow higher resolution to be attained, which is almost ~1Å better than any previously reported structure of yeast FAS.

In the structure, ~7700 water molecules were confidently modelled, representing an average of 0.33 water molecules per residue (∼0.48 waters at the central wheel, and ~0.18 waters in the dome per residue). The added resolution reveals a 22° kink in the isoalloxazine ring system of FMN in the ER active site (Figure 1B, i) that eluded observation in earlier structures. This isoalloxazine kink indicates that FMN is in its reduced form, consistent with the reductive function of ER in FA biosynthesis (Figure 1A). A hydrogen bonding network involving both side chains and water molecules stabilize FMN within the ER active site (Figure 1B, ii). Similarly, polar contacts involving both amino acid side chains and water molecules enable NADP+ recognition in both the ER and KR active sites (Figures 1B, iii and iv). The map is in agreement with a MPT active site nucleophile Ser1808-Oy malonic acid ester and a bound post-hydrolysis CoA molecule (Figure 1B, v). Owing to the lower local resolution at the MPT, very few localized water molecules could be placed.

### Visualizing ACP domains in positions representing intermediates in FA biosynthesis

All six FAS ACP domains have been observed bound to the KS domain in biochemically synchronized samples, achieved by chasing fatty acid synthesis intermediates by incubating FAS with saturating amounts of malonyl-CoA and NADPH). Starting from this "cycled" state, incubation of FAS with saturating Δγ-subunit concentrations has afforded AT-bound ACP domain stabilization with FAS domes in the rotated conformation, Singh, K., et.al., (2020). These two ACP states were observed irrespective of the symmetry imposed in cryo-EM reconstructions. However, in the 1.9Å FAS structure (Figure 1), ACP domains were poorly defined and essentially unresolved. It is hypothesized that this is a consequence of biochemical heterogeneity within the FAS dome, in the form of ACP domains occupying non-identical positions in each functional compartment. Should this indeed be the case, one would either expect that no/fragmented ACP densities are observed similar to the situation in the high-resolution FAS structure, or that more than three partial ACP densities are observed in a dome. The ACP domain is an integral part of the alpha-subunit, and thus, biochemically no more than three can exist in a FAS dome. Both scenarios would indicate imprecise averaging of single particle images in image processing, when reconstructing the FAS structure. Overcoming these challenges of biochemical heterogeneity, combined with solutions to these technical cryo-EM challenges would yield structural information, albeit at lower resolution, of interesting functional intermediates in the FA biosynthetic cycle. In the following we describe how we have overcome these image processing challenges.

Non-uniform ACP binding to various active sites within a single FAS dome most likely is the reason why ACP remain poorly resolved. To verify this, we first determined the FAS structure (without imposing symmetry) which had not been chased by malonyl-CoA and NADPH ("uncycled", referred to as "FASx" hereafter). We expected ACPs to be most randomly distributed within the FAS dome in this case. In agreement, the FASx structure displayed nine low-threshold, partial ACP densities per dome or eighteen per FAS adjacent to AT, MPT, and KS. Asymmetric structures of cycled FAS incubated with acetyl-CoA, malonyl-CoA, and NADP+ (hereafter referred to as FASamn), displayed six partial ACP densities per dome or twelve per FAS juxta-posed to KR and KS (Figure 2B, middle). Cycled, asymmetric FAS structures with acetyl-CoA and malonyl-CoA (hereafter referred to as FASam) revealed six partial ACP densities per dome or twelve per FAS residing next to ER and KS.

Visualizing more than three ACP domains within FAS domes as discussed, indicates an averaging-out effect of protein densities by image processing. In FAS, the larger, pseudo-symmetric barrel structure appears to mainly drive image alignments, and not ACP domains, which leads to this ACP density loss in reconstructions. To overcome this obstacle, a strategy involving initial 3D refinement imposing strict D3 symmetry, followed by symmetry expansion and particle signal subtraction was pursued to in-silico extract the six αβ dimers (asymmetric unit) from each FAS particle. FAS asymmetric units were then classified using masks representing a single ACP position, followed by classification without any mask. This resulted in asymmetric unit structures of ~3Å resolution, revealing full, single-position ACP densities, representing intermediate stages in FA biosynthesis.

### FAS Conformations and modes of ACP binding to enzymatic domains

FAS β-subunits have been previously shown to adopt two major conformations, non-rotated and rotated. In FASx and FASam datasets, two additional hybrid conformational states were identified. In the semi non-rotated (SNRot) state, AT and ER domains are in a non-rotated conformation, whereas DH and MPT domains adopt rotated conformations. The semi rotated (SRot) state is characterized by a largely rotated conformation, with the AT domain not fully rotated. The ACP was found AT domain-adjacent in all four β-subunit conformations which can be explained by its ability to pivot about Ser180 corresponding to aa 44 of SEQ ID No.7. This ACP swivel allows it to adapt to the space between AT and ER in different conformations, while maintaining interactions through canonical and structural domains. ACP positioned at KS and ER was correlated with non-rotated and SNRot conformations only, consistent with small changes in both domains in these conformations. ACP-KR binding, however, only correlated with non-rotated conformations. In addition, DH-bound ACP, was found in the non-rotated state. These findings suggest that the rotated β-subunit conformation is exclusive to the acetyl loading step, while the condensation- and elongation phases appear to occur in the non-rotated conformation. Therefore, these new conformations are likely adopted when ACP is in transition from AT to KS and vice-versa.

The snapshots captured herein, and in earlier studies, see Singh, K., et.al., (2020), Leibundgut, M., et.al., (2007), Science 316, 288-290. 10.1126/science.1138249, Lou, J.W., and Mazhab-Jafari, M.T. (2020), Commun. Biol. 3. 10.1038/s42003-020-0997-y, allow addressing which determinants are exploited by FAS enzymatic domains to recognize the ACP. The ACP canonical domain which includes Ppant-Ser180 corresponding to aa44 of SEQ ID No.7 and its flanking alpha-helices 2 and 3, is positioned at the active site cleft openings of MPT, KR, and ER domains (Figure 3). ACP canonical domain recognition appears predominantly electrostatic, consistent with the charge-complementarity of the interaction surfaces. These interactions resemble ACP canonical domain KS, AT, and DH domain associations and therefore appear to be universally conserved in ACP canonical domain-active site interactions. For MPT-, KR-, and ER-bound ACP, we mapped additional ACP-FAS interactions (Figure 3): ACP also contacts nearby structural elements of AT and ER through ACP helix α4 at the MPT (Figure 3, i and ii). At the ER, residues of ACP α-helices 2 and 3 along with the loop joining helices α3/4 form extensive contacts, the nearby structural elements of the AT and KS interact with ACP helices α2 and α4, respectively (Figure 3, iii and iv). At the KR, ACP helices α2 and α3 contact KR residues outside the active site, whereas the loop between ACP helices α3/4 recognizes nearby structural elements of the KR, and ACP helix α4 interacts with the KS (Figure 3, v and vi). Unlike ER and MPT, the KR-bound ACP forms additional contacts with the KR domain coiled-coil dimerization segments using its yeast-specific extension (helix α5, loop between helices α5/6 and helices α7/8).

Thus, findings in Figure 3 along with those previously reported, see Singh, K., et.al., (2020), Leibundgut, M., et.al., (2007), Science 316, 288-290. 10.1126/science.1138249, Lou, J.W., and Mazhab-Jafari, M.T. (2020), Commun. Biol. 3. 10.1038/s42003-020-0997-y infer that structural elements distant from active sites are generally involved in ACP binding. Further, the yeast-specific ACP structural domain mediates interactions when bound to KS, KR, and AT. The canonical ACP domain, however, is universally required for interaction with all enzymatic domains of FAS.

### Structural changes to accommodate the Ppant-arm in MPT, KR, and ER domains

Next, the question if local conformational adaptations of MPT, KR and ER domains aid in ACP, and in particular Ppant-arm, recognition has been addressed. The inventors have been able to capture two different variations of the Ppant-group bound to MPT: the first stems from malonyl-CoA as an isolated molecule, the other where the Ppant-group is covalently attached to the ACP Ser180 corresponding to aa 44in SEQID No. 7. In both cases, a large conformational change is observed, characterized by a movement of the MPT helical subdomain towards the MPT catalytic Ser1808 corresponding to aa 244 in SEQ ID No.6 (Figure 4A). The result is the enclosure of Ppant in a clamp-like manner. The overall MPT active site cavity expands in the ACP-bound state and could explain how malonyl-CoA or malonyl-ACP are distinguished from longer ACP-bound acyl intermediates (Figure 4B, right). At present, this explains the accommodation of Ppant-acyl adducts of up to 10 carbon atoms (~16Å), but leaves open how palmitoyl- or stearoyl-acyl adducts could bind.
Similarly, two scenarios of the NADP+-bound KR domain were captured in this manuscript: in the absence of an ACP-bound Ppant-arm (Figure 1B, iv), and in its presence (Figure 3). In both scenarios a closure of a loop on the nicotinamide-ribose moiety of NADP+ was observed (Figure 4C), similar to what has been reported previously, see Jenni, S., Leibundgut, et.al., (2007), Science 316, 254-261. 10.1126/science.1138248. The loop closure creates a pocket that represents an entry site for the ACP-bound Ppant-arm, and fulfils a dual purpose: stabilization of NADPH/NADP+ binding and the ACP-bound Ppant-arm (Figure 4C). NADPH/NADP+-binding also causes the expansion of a constriction lined by Asn829, Phe883, Leu930 and Leu936 corresponding to aa 502, 327, 374, and 380 of SEQ ID No.5, allowing the KR active site to accommodate fatty acyl intermediates longer than 4-6 carbon atoms (Figure 4D).

In the ER, the Ppant-arm enters the active site through a cleft within the FAS dome. Strikingly, the Ppant-arm is kinked by ~96° in the ER active site (Figure 4E), allowing the enoyl bond to be positioned between the catalytic His740 corresponding to aa 184 of SEQ ID No.1 and FMN. The present resolution of the structure, does not allow distinguishing a cis- or trans-enoyl bond, but both could be accommodated in this position. Superposition of the ACP-bound ER domain (Figure 3) with the NADP+-bound ER domain (Figure 1D, iii), reveals that nicotinamide and the enoyl-ACP Ppant-arm adduct would occupy the same position adjacent to the FMN isoalloxazine (Figure 4E). Accompanying this, when ACP-bound, an ER loop segment constricts the adenosine diphosphate phosphoribosyl (ADPR) binding cleft by ~3Å, making NADPH/NADP+ and enoyl-acyl-ACP binding mutually exclusive (Figure 4F).
Taken together, an ACP-Ppant-mediated motion in the MPT, and a NADPH/NADP+-mediated loop closure in the KR, result in a clamping motion that allows Ppant-arm recognition, and the accommodation of covalent acyl intermediates of ever-increasing length in FA biosynthesis. In contrast, a pre-formed cleft is exploited in the ER for the positioning of a kinked enoyl-Ppant-arm over the isoalloxazine ring of FMN, which constricts the active site to render enoyl-acyl-ACP binding mutually exclusive to NADPH/NADP+.

### Comparison of ACP-FAS enzyme interactions in type I and type II systems

The elucidation of yeast ACP (yACP) domain structures bound to several yeast FAS (yFAS) enzymatic domains hearein, allow for the comparison of ACP recognition by FAS enzymes in type I and type II systems. For this, the structure of ACP bound to the Malonyl-CoA-acyl carrier protein transacetylase (MCAT) enzyme (PDB ID: 6U0J) with yACP-bound yFAS-AT (Figure 5A), and yACP-bound yFAS-MPT (Figure 5B) structures were compared, as the MCAT serves both transacylase functions in bacteria. Further, the FabB ketosynthase structure bound to ACP (PDB ID: 6OKC) is compared to the yACP-bound yFAS-KS (PDB ID: 6QL9, (Figure 5C)), and the FabB dehydratase bound to ACP (PDB ID: 4KEH) is compared to the yACP-bound yFAS-DH (PDB ID: 6WC7, Figure 5D).

The bacterial ACP, AcpP, interacts with MCAT through α-helix 2 (corresponds to α-helix 3 in yACP) and the alpha2/3 loop, the interaction interface yielding a buried surface area (BSA) of 459 Å2 (Figures 5A and 5B). In yFAS-AT, additional structural segments exist in addition to the conserved catalytic domain (Figure 5A, I left) . Although the Ppant-Serine is similarly positioned at the opening of the active site cleft in bacterial and yFAS structures(Figure 5A, i right), the AcpP-like binding pose is not possible in the yFAS-AT due to clashes with the yFAS-AT structural loop, and adjacent ER domains segments (Figure 5A, iii). Instead, yACP alpha-helices 2 and 3 (BSA 530 Å2) interact with the AT catalytic domain, the yACP loop alpha3/4 contacts adjacent ER segments (BSA 240 Å2), and yACP alpha-helix 5 associates with the AT structural domain (BSA 263 Å2) in yFAS-AT. Thus, the yACP:yFAS-AT interaction interface has nearly doubled in comparison to MCAT-AcpP.

The yFAS-MPT also has structural segments that are additional to the catalytic domain(Figure 5B, i). yACP interacts with yFAS-MPT through its alpha-helix 3 (BSA 238 Å2), and its alpha-helices 4 and 5 to adjacent structural segments of ER- and AT-domains (BSA 216 Å2) (Figure 5B, iii). Thus, the interaction surface of yACP has redistributed with regards to AcpP to incorporate the yACP structural domain and exploit neighboring segments of the yFAS architecture, while maintaining a similar interaction interface magnitude.

Both bacterial and yFAS KS are homodimers, the latter containing additional structural segments (Figure 5C, i). AcpP interacts through its alpha-helices 2 and 3 with FabB (BSA 803 Å2). The yACP exploits alpha-helix 3 in a different pose than AcpP alpha-helix 2 to interact with the KS catalytic domain (BSA 377 Å2), and uses alpha-helices 5 and 6 to interact with KS structural segments (BSA 477 Å2). Yet again, the magnitude of the yACP interaction interface is maintained in comparison to AcpP, while employing the yACP structural domain and KS structural segments.

FabA forms a dimer, while yFAS-DH has pseudo-dimeric organisation (Figure 5D, i). AcpP α-helix 2 is primarily involved in FabA binding (Figure 5D, ii), similarly yACP α-helix 3 is primarily involved in DH interaction (Figure 5D, iii). However, as expected by the architectural differences between yFAS-DH and FabA, the yACP binding pose at yFAS-DH is distinct to AcpP FabA binding. Most likely this different binding pose is due to DH structural elements and the adjacent ER domain.

No AcpP structures exist bound to bacterial KR- and ER- enzymes, we have therefore restricted ourselves to comparing the overall architecture and co-substrate binding to the fungal-like ER FabK (PDB ID: 4IQL, Figure 6A), and KR FabG (PDB ID: 1Q7B, Figure 6B). yACP interacts with yFAS-ER mainly through its canonical domain (BSA 701 Å2), and some sparse contacts to its structural domain (BSA 69 Å2). Both FabK and yFAS-ER have an extended active site cleft with two entrances A and B (Figure 6A, i and ii). In yFAS-ER NADPH/NADP+ enters the active site cleft through entrance B (Figure 6A, iii), whereas the yACP Ppant-arm enters through entrance A (Figure S6A, iv). Considering that the ADP moiety of NADPH/NADP+ adopts a similar position at entrance B in FabK as in yFAS-ER (Figure 6A, v, and vi), and a competitive inhibitor of FabK occupies the Ppant-arm binding site at entrance A (Figure 6A, vii, and viii), ACP recognition is likely to be conserved in both systems.

FabG is tetrameric, while the yFAS-KR dimerises and the catalytic fold between type I and type II systems is well conserved (Figure 6B, i and ii). NADP+-bound FabG (Figure 6B, iii) resembles apo yFAS-KR (Figure 6B, iv) which is much more open than the NADP+-bound yFAS-KR (Figures 4C and 6B, v). As NADP+ and the catalytic tyrosine residue superpose well in both FabG and yFAS-KR, the AcpP-Ppant-arm in FabG could occupy a similar position as in yFAS-KR (Figures 4C and 6B, vi). Further, the FabG active site cleft is more open and could easily accommodate long fatty acyl chains.

### Exploitation of ACP structural snapshots in the FA biosynthesis cycle to re-engineer FAS

Structures of yACP bound to several yFAS enzymatic domains potentially provide a route to re-engineer the FA biosynthetic output of yFAS. Importantly, mapping the Y. lipolytica (an oleaginous, biotechnologically relevant yeast) FAS sequence onto the structural snapshots obtained in this manuscript, reveals that all ACP and enzymatic domain amino acids involved in mutual interaction are conserved. The free internal volume within the FAS barrel considering all ACP positions and yFAS conformations is estimated to be ~3.9 x 105 Å3, reduced to ~ 3.6 x 105 Å3 by binding of 6 γ-subunits, suggesting that each yFAS dome can accommodate up to about 150 kDa (300 kDa for total FAS) of additional exogenous globular proteins. As the γ-subunit resides with the FAS dome and binds in a specific manner it can be exploited as a vector.

The inventors performed a proof-of-concept experiment to address the feasibility of this (Figure 6A). Δγ-FAS was reconstituted with either the γ-subunit alone, or the following proteins replacing γ-subunit residues 114 - 132: 1 copy of the fluorescent protein UnaG (y-1xUnaG; 30 kDa), 2 head-to-tail copies of UnaG (γ-2xUnaG; 45 kDa), V. harveyi thioesterase TesA (γ-Tes; 50 kDa), or V. harveyi TesA not incorporated into the γ-subunit scaffold as a control (Tes). Due to the widely differing molecular weights of these proteins and the 2.6 MDa yFAS, the reconstitution mixture was fractionated by sucrose-density centrifugation. Proteins co-sedimenting with yFAS in high-molecular weight fractions were assumed to be bound. All proteins, when incorporated into the γ-subunit vector bound to Δγ-FAS, but notably Tes alone did not. In the case of γ-1xUnaG, and γ-2xUnaG, substantial increase relative to the intrinsic FMN fluorescence of Δγ-FAS was seen. Finally, by LC/MS we were able to verify that Δγ-FAS reconstituted with γ-Tes exhibited a different acyl-CoA product profile in comparison to Δγ-FAS alone, or Δγ-Δγ-FAS reconstituted with the γ-subunit. Notably, the acyl-CoA product profile of Δγ-FAS reconstituted with γ-Tes exhibited a marked higher abundance in C8-, and C10-CoA and a lower abundance in C12-, C14-, and C16-CoA. As a Δγ-FAS reaction subsequently treated with V. harveyi TesA displayed a similar product profile, we attribute this product profile change to the thioesterase specificity within the FAS dome. Together, the inventors have demonstrated that knowledge about yACP structural snapshots can inform about ways to modulate yFAS product profiles in oleochemical production.

### Discussion

Herein, the inventors have elucidated enzymatic domain-bound ACP snapshots, representing functional intermediates in the yeast FA biosynthesis cycle. These include AT-, KS-, MPT-, KR-, and ER-bound ACP states, as well as substrate-, cosubstrate-, and cofactor-bound states. For AT-, MPT-, KR-, and ER-bound ACP domains, we were able to resolve the functionally essential Ppant-arm which enables the shuttling of starter chemical groups, biosynthetic intermediates, and products as covalent adducts from one active site to the other. This has allowed us to investigate modes of ACP- and Ppant-arm recognition in distinct functional states and structural consequences thereof. Together, the reported findings, along with those previously reported, see above, allows us to reconstruct a complete architectural framework of transitions in the FA biosynthetic cycle (Figure 7A).

These snapshots suggest that each FAS dome could accommodate exogenous proteins, 150 kDa in mass. Proof-of-concept experiments show that such exogenous proteins could be used to modulate FAS product profiles. This framework represents the solid foundation to interrogate FA biosynthesis mechanisms at atomic resolution, develop activity-based and biophysical probes, aid in rational inhibitor design, as well as modify, and exploit its function in oleochemical production.

### High resolution structure determination of type I FAS using cryo-EM

A pre-requisite to gain functional, mechanistic insight and establish structure-function relationships, is a detailed and accurate description of enzyme active sites, and their structural change in functional states. This entails the protein backbone and side-chains, prosthetic groups, co-substrates, and the ordered solvent (waters and ions) of the macromolecular complex. While all these features are identifiable at different resolution thresholds, ordered solvent can be accurately described at better than 2.2Å. Particularly in cryo-EM, many factors represent impediments to attain such detailed structures: biochemical, compositional and conformational heterogeneity, heterogeneity arising from cryo-EM grid preparation, technical EM imaging limitations, and pitfalls arising from low signal to noise image processing.

For S. cerevisiae FAS, we have tackled biochemical heterogeneity by establishing a mild chromatography-free purification procedure that allows FAS structure determination by cryo-EM and X-ray crystallography to better than 3 Å.11 We discovered a weakly bound γ-subunit of yFAS that readily dissociates in the concentrations used for cryo-EM grid preparation. To overcome this, we purified FAS from an S. cerevisiae strain with a genetic deletion for the γ-subunit. We additionally added saturating concentrations of malonyl-CoA and NADP+, to lock the respective enzymatic domains in uniform compositional states. For cryo-EM structure determination, we used a 300kV Titan Krios Mono/BCOR microscope with optimized electron optics 14. In the case of yFAS, this has led to structure determination at a global resolution of 1.9Å, an experimental B-factor of 53Å2, and a local resolution spread from 1.9Å at the central wheel to 2.7Å at the top of the dome (Figure 1B). The B-factor obtained for yFAS with the 300kV Titan Krios Mono/BCOR microscope, is similar to apo-ferritin imaged with a conventional Titan G3 Krios instrument, see Danev et al. Trend Biochem Sci 44, 837-848, https://doi.org/10.1016/j.tibs.2019.04.008. This highlights that EM optical improvements aid in improving the resolution attained by almost 1 Å proportional to the previously described local resolution spread even for dynamic assemblies such as FAS. The high-resolution structure has confirmed that FMN is present in its reduced form in the ER (Figures 1B, i), provided insight into CoA recognition, and a covalent Ser1808-malonyl adduct in the MPT (Figure 1B, v), and NADPH/NADP+ recognition by protein and ordered solvent in the KR and ER (Figures 1B, ii-iv). Yet notably, the ACP domain is not resolved in the high-resolution structure (Figure 1), and is partially visible in numbers exceeding those dictated by its biochemical composition in other instances (Figure 2). This can either be explained by 1) insufficient biochemical control to synchronize in distinct functional states, and/or 2) an orientation assignment problem in image alignments due to the pseudo-symmetric dome resulting in difficulties to sort for conformational heterogeneity, independent- and asynchronous ACP motions, and failure to detect ACPs in whole FAS particles.

### Structural ACP snapshots and conformational states in the FA synthesis cycle:

Standard 3D classification procedures were unable to reveal ACP domains in full FAS. Sorting for ACP positions, and β-subunit conformations by computationally extracting FAS asymmetric units has visualized ACP snapshots representing functional intermediates in the FA synthesis cycle. Two additional hybrid β-subunit conformations representing transitions from rotated to non-rotated conformations were identified. An inventory of β-subunit conformations and corresponding ACP positions, suggests that the rotated state is exclusively reserved for the AT-bound ACP position. The AT-bound ACP is also compatible with SRot, SNRot, and non-rotated conformations. We thus infer that catalytic AT serine loading by the starter acetyl group, is followed by ACP movement to the AT domain and Ppant-arm positioning to accept the acetyl group. ACP AT-binding is most likely accompanied by a non-rotated to rotated state conformational change. When transferring the acetyl group to the KS catalytic cysteine, ACP domain motion to the KS, and non-rotated state adoption must occur. These changes and ACP motions are sufficient to explain the energy barrier we had previously reported, Singh, K., et.al.,(2020), see above, in the transition from rotated to non-rotated conformations. We had previously speculated, Singh, K., et.al.,(2020), see above, that every FAS functional state would adopt a distinct β-subunit conformation. The β-subunit conformation/ ACP location survey does not confirm this. ACP binding to every other enzymatic domain occurs in the non-rotated conformation. This might avoid kinetic barriers in condensation and elongation cycles, and increase processivity.

Small scale conformational changes very much do occur in condensation and elongation cycles. For FA biosynthesis, the malonyl group must be activated by the MPT catalytic serine and transferred onto the ACP Ppant-arm (Figures 1B, v; 3, i and ii; and 4A). MPT closes when malonyl-CoA is bound, opens to release CoA, and closes again to accommodate the ACP-Ppant-arm to enable malonyl transfer. The ACP-bound MPT active site is more spacious allowing larger ACP-bound acyl chain accommodation for release, however, how palmitoyl- or stearoyl-Ppant-acyl adducts might bind remains unclear at present. The malonyl-Ppant adduct is then taken to the KS where a Claisen condensation reaction forms a β-keto intermediate. NADPH then populates the KR active site causing KR loop clamping onto NADPH (Figures 1A, and 4C), expanding the space for Ppant-arm entry, and accommodating β-keto intermediates (Figures 4C and 4D). Following reduction to a β-hydroxy intermediate, the ACP proceeds to the DH, see Lou and Mazhabi-Jafari (2020) above, to form a trans-2-enoyl intermediate. Dehydration most likely establishes the cis- or trans-configuration of the enoyl intermediate and the following regiospecificity. However, neither does the published structure resolve the Ppant-arm, nor were the present inventors yet able to observe this state in our own experiments to address this question. To complete the FA biosynthesis cycle (Figure 1A), the ACP interacts with reduced FMN in the ER where the Ppant-arm kinks, and its binding leads to the constriction of the NADPH/NADP+ binding cleft (Figure 4E). ACP Ppant-enoyl adduct, and NADPH/NADP+ binding is mutually exclusive suggesting either the NADPH/NADP+ departure before ACP arrival, or that ACP binding triggers NADPH/NADP+ release. The ACP then transfers the Ppant-fatty acyl adduct to either the KS active site cysteine for additional elongation cycles, or the MPT domain where the Ppant-acyl adduct is trans-acetylated to a CoA molecule and released. Associated with the multiple steps in which the ACP is KS-bound (transfer of acetyl, condensation of acetyl with malonyl, condensation and transfer of variety fatty acyl-lengths with malonyl), the biochemical heterogeneity appears to be too large to resolve the Ppant-arm. However, we were able to resolve the Ppant-arm both by cryo-EM and X-ray crystallography when using cycled FAS as previously reported, Singh, K., et.al.,(2020), see above. We thus hypothesize that KS Ppant-recognition might be significantly different depending on the precise nature of acyl-adduct carried by the ACP domain. A series of structures at high resolution with defined intermediates bound to the catalytic cysteine of the KS will be essential to resolve this matter.

### Larger ACP in fungal Type I FAS appears to bring order within the FAS dome

A possible logistical problem imposed by the spatial confines of yFAS architecture looms: the common use of ACP domain and its Ppant-arm could elicit promiscuity in recognition. Bacterial FAS enzyme-ACP interactions rely on a minimal interaction interface that encompasses the PPant-arm and electrostatic interactions (Figures 5 and 6). Promiscuous ACP-binding to sites in a different order than the one strictly required by the FA biosynthetic sequence would decrease catalytic efficiency. A potential resolution of this problem could be the additional structural domain of yFAS. Indeed, we observe the association of yACP structural domains and structural domains of several enzymatic domains (Figures 5, and 6). Taking all the data summarized in Figure 7A into account, we are able to conclude that the canonical yACP domain extensively contacts all active sites, and is often supplemented by interactions of the structural domain. yFAS-yACP interaction affinity, as studied by buried surface areas in interfaces, appears to be conserved in comparison to bacterial counterparts but pronouncedly distributed between yACP canonical domain-active site cleft interactions and yACP structural domain-structural segment interactions (Figures 5 and 6). Thus, ACP recognition is likely to be governed by avidity rather than affinity in type I FAS. In addition, the mode of yACP positioning at enzymatic domains suggests that the yACP structural domain may play a more active role. yACP binding by steric-hindrance is mutually exclusive between 1) AT and its proximal MPT and ER domains, 2) MPT and its proximal AT and ER domains, as well as 3) DH and its proximal ER domain (Figure 7B, ii-vi). The steric hindrance would be less pronounced in the absence of the yACP structural domain (Figure 7B, i). Notably, the "path of least resistance" for the ACP to shuttle along the FA biosynthetic sequence is common to the functional compartment we inferred by γ-subunit binding. Thus, the shuttling path least obstructed by the yACP structural domain, coincides with the shortest path required to transverse the FAS dome, ultimately increasing FA biosynthetic productivity. Another consequence of the yACP structural domain-exerted steric hindrance, is ensuring that the enzyme active site to which the ACP next transitions is empty. Therefore, in addition to minimising recognition promiscuity, the yACP structural domain may promote compartmentalisation, and ensure productive sequential order in FA biosynthesis.

## Claims

1. A protein involved in fatty acid synthesis, said protein comprises one or more polypeptide chains, wherein said polypeptide chain(s) comprise
i) one or more subunits comprising the amino acid sequence of
SEQ ID No. 1 (ER domain);
SEQ ID No. 2 (AT domain);
SEQ ID No. 3 (KS domain);
SEQ ID No.4 (DH domain);
SEQ ID No.5 (KR domain);
SEQ ID No.6 (MPT domain); and/or
SEQ ID No. 7 (ACP domain);
ii) having at least one amino acid substitution in the ER domain at a position corresponding to 32-140, 159-190, 211-220, 239-250, 288-329, 381-390, 450-480, 500-505 of the amino acid sequence of SEQ ID No. 1; and/or at least one amino acid substitution in the AT domain at a position corresponding 5-141, 162-166, 273-277, 362-368, 291-430, 542-554 of the amino acid sequence of SEQ ID No. 2; and/or at least one amino acid substitution in the KS domain at a position corresponding to amino acids 68-131of the amino acid sequence of SEQ ID No. 3; and/or an amino acid substitution in the DH domain at a position 189-310 corresponding to of the amino acid sequence of SEQ ID No. 4; and/or an amino acid substitution in the KR domain at a position corresponding to 36-66, 176-187, 353-417, 442-460, 498-520, 543-560, 602-643 of the amino acid sequence of SEQ ID No. 5; and/or at least one amino acid substitution in the MPT domain at a position corresponding to 1-30, 48-170, 242-276, 293-306, 325-335, 353-358, 396-415 of SEQ ID No. 6; and/or at least one amino acid substitution in the ACP domain at a position corresponding to 5-11, 20-25, 34-105, 135-147 of SEQ ID No. 7;
wherein the amino acid sequence comprising the at least one amino acid substitution has at least 70 %, or preferably at least 80 % or 90 % or 95 % sequence identity to the respective amino acid sequence of SEQ ID No. 1 and/or of SEQ ID. No. 2 and/or SEQ ID No. 3 and/or SEQ ID No. 4 and/or SEQ ID No. 5 and/or SEQ ID No. 6 and/or SEQ ID No. 7; resulting in altered activity of fatty acid synthesis.

2. The protein according to claim 1 comprising at least one amino acid substitution in the ER domain at a position corresponding to 40-45, 65-70, 92-109, 126-140, 160-170, 175-188, 314-324, 290-301, 467-477 of the amino acid sequence of SEQ ID No. 1; and/or at least one amino acid substitution in the AT domain at a position corresponding 163, 274, 364, 401, 403, 405, 406, 428 of the amino acid sequence of SEQ ID No. 2; and/or at least one amino acid substitution in the KS domain at a position corresponding to amino acids 95-102, 109-130 of the amino acid sequence of SEQ ID No. 3; and/or an amino acid substitution in the DH domain at a position 198 corresponding to of the amino acid sequence of SEQ ID No. 4; and/or an amino acid substitution in the KR domain at a position corresponding to 39-62, 178-184, 354-364, 372-392, 409-413, 545-557, 603-642 of the amino acid sequence of SEQ ID No. 5; and/or at least one amino acid substitution in the MPT domain at a position corresponding to 20-28, 48-64, 81-96, 102-110 of SEQ ID No. 6; and/or at least one amino acid substitution in the ACP domain at a position corresponding to 40-49, 51-70, 72-105of SEQ ID No. 7.

3. The protein according to any one of the preceding claims wherein the amino substitutions are as shown in table 1.

4. A nucleic acid molecule coding for a protein or for a polypeptide according to any one of claims 1 to 3 preferably, further comprising vector nucleic acid sequences, more preferably expression vector sequences and/or comprising promotor nucleic acid sequences and terminator nucleic acid sequences, and/or comprising other regulatory nucleic acid sequences and/or in the nucleic acid molecule preferably comprises dsDNA, ssDNA, cDNA, LNA, PNA, CNA, RNA or mRNA or combinations thereof.

5. A host cell containing a nucleic acid molecule according to claim 4 and preferably expressing said nucleic acid molecule, wherein said host cell is preferably selected from a bacterial cell, a fungus cell, and an algae cell, actinobacteria cell or eubacteria cell.

6. The host cell according to claim 5, wherein said host cell is a non-oleaginous yeasts or a oleaginous yeasts cell, preferably, said host cell is an oleaginous yeast genus selected from Candida, Rhodosporidium, Yarrowia, Cryptococcus, Rhodotorula, Lipomyces, and Trichosporon, like Yarrowia lipolytica or a non-oleaginous yeast genus selected from Saccharomyces, Schizosaccharomyces, Pichia pastoris or Kluyveromyces lactis and Ustilago maydis.

7. A method for the production of fatty acids, comprising the step of expressing a nucleic acid molecule according to claim 4, preferably in a host cell according to claim 5 or 6, or of the protein or polypeptide according to any one of claims 1 to 3.

8. The method according to claim 7 for the production of short chain (C6 to C12) fatty acids wherein the protein or polypeptide contains the following substitutions: Met56Val, Met56lle, Val26Leu, Val26Ile, Lys62Arg, Gln58Glu, Lys64Arg, Lys83Arg, Asn326Asp, Asn326Gln, Asn326Glu, Asn328Asp, Asn328Gln, Asn328Glu, Gln333His, Gln333Glu, Arg398Glu, Arg398His, His413Asp, His413Asn, His413Glu, His413Gln, His413Tyr, His413Arg in SEQ ID No.6; or Lys37Arg, Lys37Glu, Lys37Gln, Gly42his, Gly42Tyr, Gly42Phe, Asn48Asp, Asn48Gln, Asn48Glu, Asn48His, Asn48Arg, Asn48Tyr, Thr45Met, Thr45His, Thr45Asn, Thr45Asp, Thr45Gln, Thr45Glu, Leu51Arg, Leu51Tyr, Lys64Glu, Lys64Arg, Glu67Asn, Glu67Asp, Glu67Gln, Glu67His, Glu67Tyr, Ser98Tyr, Ser98His, Ser98Glu, Ser98Gln, Ser98Arg, Ser98Lys, Ser99Tyr, Ser99His, Ser99Glu, Ser99Gln, Ser99Arg, Ser99Lys in SEQ ID No.7, or expressing a nucleic acid molecule coding for said protein or polypeptide, preferably in a host cell according to any one of claims 5 to 7.

9. A use of a protein according to any one of claims 1-3, a nucleic acid according to claim 4 or a host cell according to claim 5 or 6 for:
the production of biofuels;
the production of fine chemicals;
the determination of actinobacterial biocides;
the determination of agents that reduce actinobacterial mycolic acid production;
the determination of fungicides.

10. An *in-silico* method of identifying molecules capable of selectively altering a step of fatty acid synthesis by FAS I comprising determining and modelling molecules interacting under physiological conditions with at least one amino acid in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131 of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7.

11. An *in-silico* method of identifying molecules capable of selectively altering the activity of an enzymatic domain present in the FAS whereby the molecules determined and modelled show interaction under physical conditions with at least one amino acid as in the ER domain at a position corresponding to 32-140, 159-190, 211-220, 239-250, 288-329, 381-390, 450-480, 500-505 of the amino acid sequence of SEQ ID No. 1; and/or at least one amino acid in the AT domain at a position corresponding 5-141, 162-166, 273-277, 362-368, 291-430, 542-554 of the amino acid sequence of SEQ ID No. 2; and/or at least one amino acid in the KS domain at a position corresponding to amino acids 68-131of the amino acid sequence of SEQ ID No. 3; and/or an amino acid in the DH domain at a position 189-310 corresponding to of the amino acid sequence of SEQ ID No. 4; and/or an amino acid in the KR domain at a position corresponding to 36-66, 176-187, 353-417, 442-460, 498-520, 543-560, 602-643 of the amino acid sequence of SEQ ID No. 5; and/or at least one amino acid in the MPT domain at a position corresponding to 1-30, 48-170, 242-276, 293-306, 325-335, 353-358, 396-415 of SEQ ID No. 6; and/or at least one amino acid in the ACP domain at a position corresponding to 5-11, 20-25, 34-105, 135-147 of SEQ ID No. 7; resulting in altering at least enzymatic activity in the FAS.

12. A method of identifying a compound capable of altering at least one of the enzymatic activity of FAS, said method comprising bringing into contact a candidate compound with the FAS and determining whether said candidate compound (i) interact with at least one amino acid as defined in any one of claims 10 or 11; and/or (ii) Is a competitor of ACP in interacting with at least one amino acid with at least one amino acid as defined in any one of claims 10 or 11.

13. A protein having mutations of at least one amino acid at a position corresponding to 5-11, 20-25, 34-105, 135-147 of SEQ ID No. 7 or not containing the amino acid sequence of 1 to 79 of SEQ ID No. 7, preferably, having mutations of at least one amino acid substitution in the ACP domain at a position corresponding to 40-49, 51-70, 72-105 of SEQ ID No. 7 or consisting of amino acids 80 to 169 of SEQ ID No.7.

14. The protein according to claim 13 for use as a fungicide or actinobacterial biocide or for use in inhibiting the enzymatic activity of at least one enzyme present in fungal FAS.

15. The method according to any one of claims 11 to 13 for determining a fungicide.
